# EUROPEAN PATENT APPLICATION

(11) **EP 4 310 193 A1**
(43) Date of publication of application: **24.01.2024**
(21) Application number: 23186477.8
(22) Date of filing: 19.07.2023
(51) Int. Cl.: C12Q 1/26, C12Q 1/00, A61B 5/00, A61B 5/145

(54) **ACRYLATE HYDROGEL MEMBRANE FOR DUAL FUNCTION OF DIFFUSION LIMITING MEMBRANE AS WELL AS ATTENUATION TO THE FOREIGN BODY RESPONSE**

(30) Priority: 20.07.2022 US 202263390896 P; 29.06.2023 US 202318344273
(71) Applicant: MEDTRONIC MINIMED, INC., Northridge, CA 91325-1219 (US)
(72) Inventor: Pesantez, Daniel E., Northridge, 91325-1219 (US); Rao, Ashwin K., Northridge, 91325-1219 (US); Somasuntharam, Inthirai, Northridge, 91325-1219 (US)
(74) Representative: Zimmermann & Partner Patentanwälte mbB

(57) **Abstract**

Embodiments of the invention provide compositions useful in implantable devices such as analyte sensors as well as methods for making and using such compositions and devices. In typical embodiments of the invention, the device is a glucose sensor comprising an analyte modulating layer formed from acrylate hydrogel composition that modulates the diffusion of glucose through the analyte modulating layer and which further comprises a bioactive agent selected to enhance the biocompatibility of analyte sensors when implanted *in vivo.*

## Description

### CROSS-REFERENCE TO RELATED APPLICATIONS

This application claims priority under Section 120 from U.S. Patent Application Serial No. 63/390,896, filed July 20, 2022, the contents of which are incorporated herein by reference.

### BACKGROUND OF THE INVENTION

### 1. Field of the Invention.

This invention relates to compositions and methods useful for implantable devices such as analyte sensors.

### 2. Description of Related Art.

A wide variety of medical conditions are treated by introducing implantable medical devices into an *in vivo* location within a human patient. However, when such devices are introduced into and/or manipulated *in vivo*, the proximal *in vivo* tissue can be disturbed or injured, leading to immune responses, clot formation and/or thrombosis at the site of implantation. Moreover, if the medical device is left within the patient for an extended period of time, thrombus often forms on the device itself, again causing fibrosis, stenosis or occlusion.

There is a need in the art for improved compositions and methods that can be used with implantable medical devices such as implantable glucose sensors. In particular, there is a need for sensor compositions and methods that can deliver drugs or other bioactive materials *in vivo* during or following a medical procedure in order to modulate the immune response at the site of implantation. Embodiments of the invention disclosed herein satisfy this need.

### SUMMARY OF THE INVENTION

Embodiments of the invention provide acrylate hydrogel compositions useful in analyte sensors as well as methods for making and using such compositions in analyte sensor membranes. Typical embodiments of the invention include an amperometric glucose sensor comprising an analyte modulating membrane (e.g., glucose limiting membrane) formed from an acrylate hydrogel and comprising a bioactive agent selected to provide such analyte sensors with improved material properties such as enhanced biocompatibility (e.g., by inhibiting a foreign body reaction (FBR)). As disclosed herein, when these acrylate hydrogel membranes are used in amperometric glucose sensors implanted *in vivo*, the resultant sensors exhibit improved sensor longevity as compared to control sensors having compositions formed from the same materials without the bioactive agents. As discussed below, embodiments of the invention also include methods designed to streamline analyte sensor production by minimizing manufacturing steps, processing time and sensor design complexity.

The invention disclosed herein has a number of embodiments. One embodiment of the invention is an amperometric analyte sensor comprising a working electrode having a selected constellation of layered elements. Typically, these elements comprise a base layer; a conductive layer disposed over the base layer; an analyte sensing layer disposed over the conductive layer; and an analyte modulating layer disposed over the analyte sensing layer. In such embodiments, the analyte modulating layer is formed from materials that allow this layer to permselectively modulate the diffusion of glucose and oxygen therethrough such that the diffusion of glucose is limited relative to oxygen; and is formed from an acrylate hydrogel having a polymer reversibly coupled to a bioactive agent so that the bioactive agent is uncoupled from the polymer in response to a stimulus. Typically in these embodiments, the analyte modulating layer exhibits a permeability to glucose and oxygen that is altered by less than 10% following release of the bioactive agent from the analyte modulating layer.

In certain embodiments of the invention, the amperometric analyte sensor consists of a single sensor flex assembly comprising a flexible planar element having a longitudinal member comprising a first side and a second side upon which the working electrode (coated with the various layers of materials disclosed herein) is disposed. In embodiments of the invention, the bioactive agent can be mixed within, or alternatively coupled on an external surface of the analyte modulating layer, and comprises at least one of: an antibacterial agent, an anti-inflammatory agent and an anticoagulant agent (e.g., at least one of a dexamethasone, a heparin or a fluoroquinolone). In typical embodiments of the invention, the bioactive agent is noncovalently entrapped within the polymer; and/or the bioactive agent is covalently coupled to the polymer (e.g., where the bioactive agent is coupled to the polymer by an acrylate moiety disposed on the bioactive agent). Typically, the analyte modulating layer comprises at least one of: a Poly(2-hydroxyethyl methacrylate), a polyurethane and a chain extender.

In illustrative embodiments of the invention, the bioactive agent is uncoupled from the polymer within the analyte modulating layer in response to: exposure to aqueous media; and/or exposure to glucose. In some embodiments of the invention, the bioactive agent is uncoupled from the polymer in response to an alteration in the pH of the environment in which the amperometric analyte sensor is disposed. In other embodiments of the invention, the bioactive agent is uncoupled from the polymer in response to an alteration in the temperature of the environment in which the amperometric analyte sensor is disposed. In other embodiments of the invention, the bioactive agent is uncoupled from the polymer in response to an electrochemical stimuli (e.g. by modulating the bias of the applied potential, by modulating the current density, by using continuous or pulsed conditions and the like).

Embodiments of the invention also include methods of making an amperometric analyte sensor electrode for implantation within a mammal. Typically, such methods include the steps of forming a working electrode comprising: a base layer; a conductive layer formed on the base layer; an analyte sensing layer disposed on the conductive layer; and an analyte modulating layer formed on the analyte sensing layer. In such embodiments, the analyte modulating layer is formed from materials selected to permselectively modulate the diffusion of glucose and oxygen therethrough such that the diffusion of glucose is limited relative to oxygen; and the analyte modulating layer is formed to comprise an acrylate hydrogel having a polymer reversibly coupled to a bioactive agent such that the bioactive agent is uncoupled from the polymer in response to a stimuli. In these methods, bioactive agent is typically selected to be at least one of: an antibacterial agent, an anti-inflammatory agent and an anticoagulant agent (e.g., a dexamethasone, a heparin or a fluoroquinolone). In certain embodiments of the invention, the bioactive agent is formed to comprise an acrylate moiety which couples the bioactive agent to the polymer. Typically, the analyte modulating layer comprises at least one of: a Poly(2-hydroxyethyl methacrylate), a polyurethane and a chain extender. In certain embodiments of the invention, the analyte modulating layer is formed from a reaction mixture comprising a photoactive agent selected to facilitate polymerization.

In some embodiments of these methods, the bioactive agent is formed to be noncovalently entrapped within the polymer. In other embodiments of the invention, the bioactive agent is formed to be covalently coupled to the polymer (e.g., where a bioactive agent is coupled to the polymer by an acrylate moiety disposed on the bioactive agent). Optionally, the bioactive agent is coupled to an external surface of the analyte modulating layer. In certain embodiments, the method comprises forming the sensor electrode from materials selected to modulate the hydrophilicity of the analyte modulating layer when the sensor electrode is disposed in an interstitial space. In some methods of the invention, the bioactive agent is formed in the sensor so that it is uncoupled from the polymer in response to an alteration in the pH of the environment in which the amperometric analyte sensor is disposed. In other embodiments of the invention, the bioactive agent is formed in the sensor so that it is uncoupled from the polymer in response to an alteration in the temperature of the environment in which the amperometric analyte sensor is disposed. In other embodiments of the invention, the bioactive agent is formed in the sensor so that it is uncoupled from the polymer in response to an electrochemical stimuli. Some methods of the invention further comprise performing a sterilization step on the sensor electrode, wherein the sterilization step comprises exposure to ethylene oxide.

Yet another embodiment of the invention is a method of sensing an analyte such as glucose within the body of a mammal using an electrochemical analyte sensor while simultaneously inhibiting a foreign body response to the electrochemical analyte sensor, the method comprising: implanting an electrochemical analyte sensor disclosed herein into the mammal; sensing an alteration in current at the working electrode in the presence of the analyte; and then correlating the alteration in current with the presence of the analyte, so that the analyte is sensed.

Other objects, features and advantages of the present invention will become apparent to those skilled in the art from the following detailed description. It is to be understood, however, that the detailed description and specific examples, while indicating some embodiments of the present invention are given by way of illustration and not limitation. Many changes and modifications within the scope of the present invention may be made without departing from the spirit thereof, and the invention includes all such modifications.

Further disclosed herein are embodiments of the invention providing compositions useful in implantable devices such as analyte sensors as well as methods for making and using such compositions and devices. In typical embodiments of the invention, the device is a glucose sensor comprising an analyte modulating layer formed from acrylate hydrogel composition that modulates the diffusion of glucose through the analyte modulating layer and which further comprises a bioactive agent selected to enhance the biocompatibility of analyte sensors when implanted *in vivo.*

### BRIEF DESCRIPTION OF THE FIGURES

The figures show illustrative aspects and embodiments of the invention.

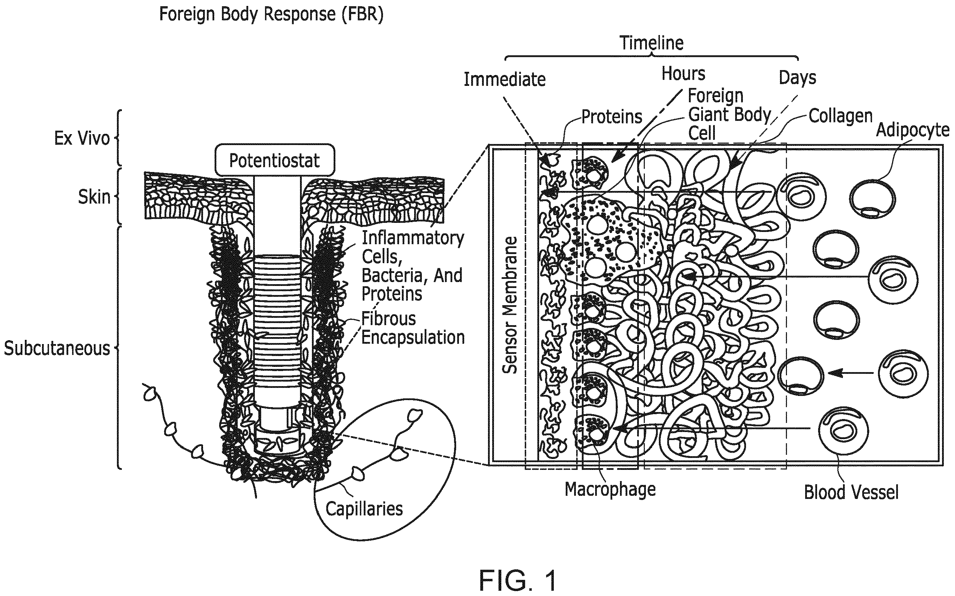

**Figure 1** provides a cartoon schematic showing foreign body responses to a sensor implanted in an interstitial space of an individual (PRIOR ART, see e.g. Nichols et al., Chem. Rev. 2013, 113, 2528-2549).

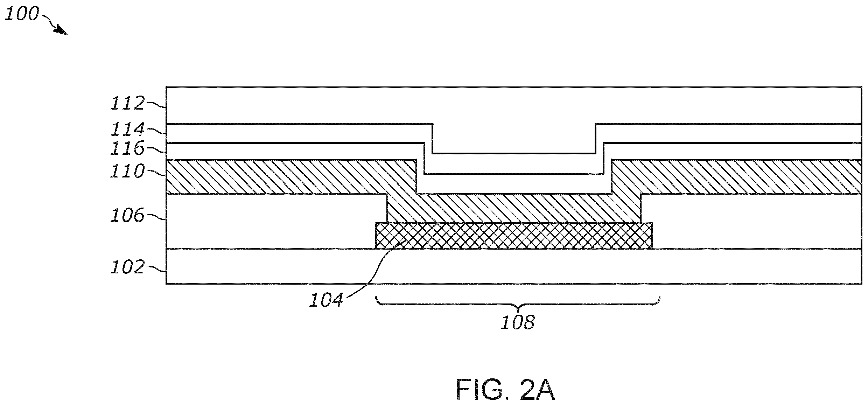

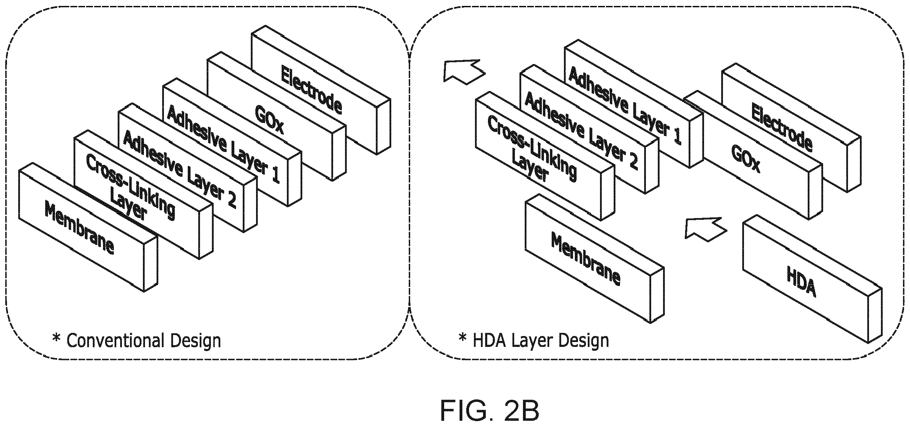

**Figures 2A-2B** provide schematics showing a conventional (PRIOR ART) sensor design comprising an amperometric analyte sensor formed from a plurality of planar layered elements which include albumin protein layer and an adhesion promoter layer (Figure 2A); and a schematic showing differences between such conventional multilayer sensor stacks and sensor stacks having a high density amine layer (Figure 2B).

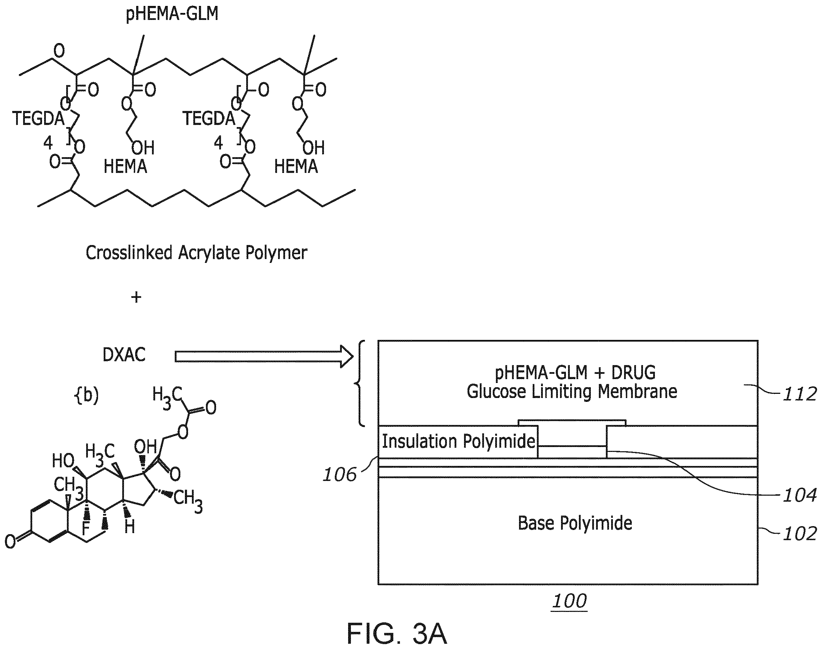

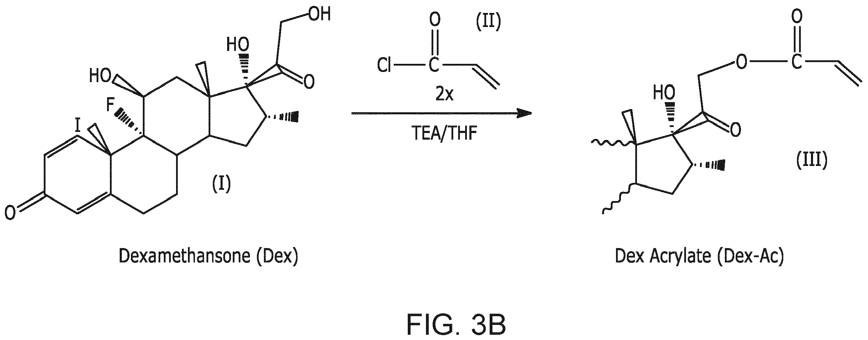

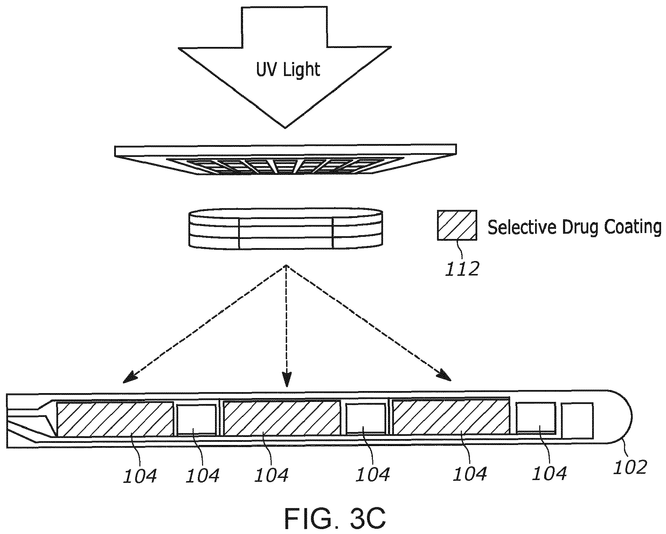

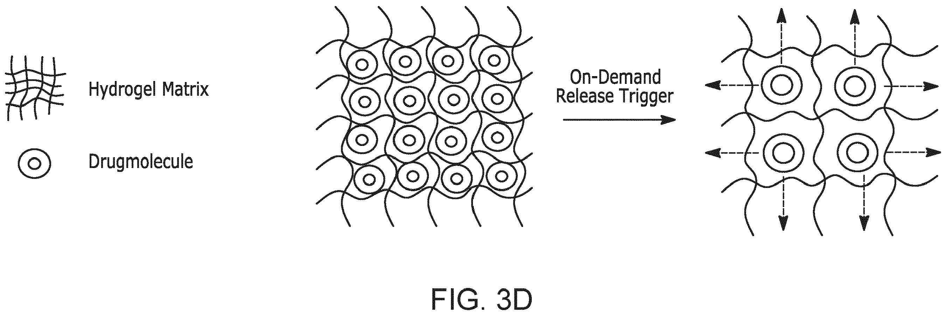

**Figures 3A-3D** provide cartoon illustrations of sensor elements and sensors of the invention. FIG 3(A) shows schematics of an analyte modulating layer material comprising a Poly(2-hydroxyethyl methacrylate) glucose limiting membrane (GLM) combined with dexamethasone acetate (left panel) and a cartoon of a illustrative sensor structure (right panel) comprising this analyte modulating layer material. FIG 3(B) shows a cartoon of a schematic for functionalizing dexamethasone with an acrylate moiety. FIG. 3(C) shows a cartoon of a schematic of a method for making sensor embodiments of the invention using light mediated polymerization/curing of sensor layer materials. FIG. 3(D) shows a cartoon of a hydrogel matrix comprising a drug molecule bioactive agent and a manner in which this bioactive agent can be released on demand.

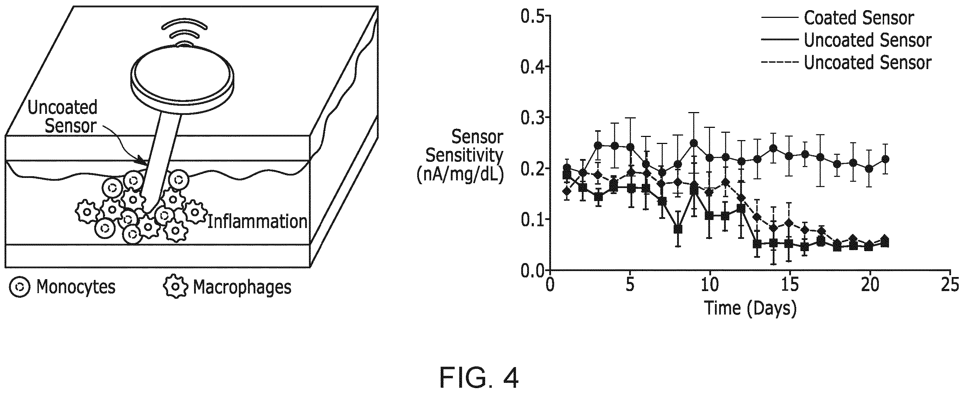

**Figure 4** provides a cartoon schematic showing foreign body responses to a sensor implanted in an interstitial space of an individual (left panel) and data from a study of sensor stability (right panel) of sensors coated with a bioactive agent (dexamethasone) as compared to uncoated sensors. The graphed data study of sensor stability (right panel) shows changes in sensor sensitivity in uncoated versus coated sensors (i.e., sensors coated with the analyte modulating layer comprising an acrylate hydrogel having a polymer reversibly coupled to a bioactive agent such that the bioactive agent is uncoupled from the polymer in response to a stimuli) over a period of about three weeks. As shown in this right panel, coated sensors retain sensitivity over time as compared to uncoated sensors.

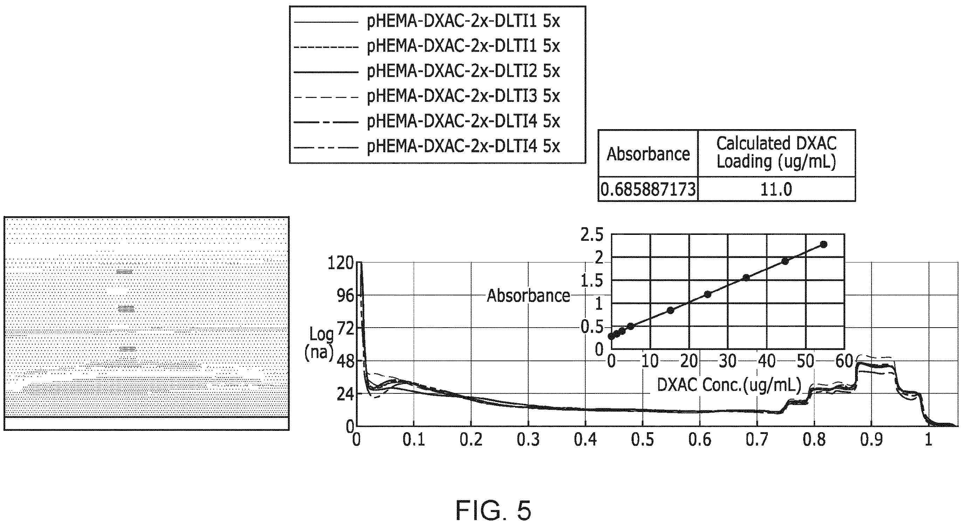

**Figure 5** provides a photomicrograph (left panel) of a layered sensor structure (left panel) and data from a study of sensor stability (right panel) of sensors coated with a bioactive agent (dexamethasone) as compared to uncoated sensors. The graphed data (right panel) is from a study of (coated sensor) signals (Isig) over time in response to analyte. The superimposed graph in the middle of this panel shows the elution of dexamethasone from sensor materials over time.

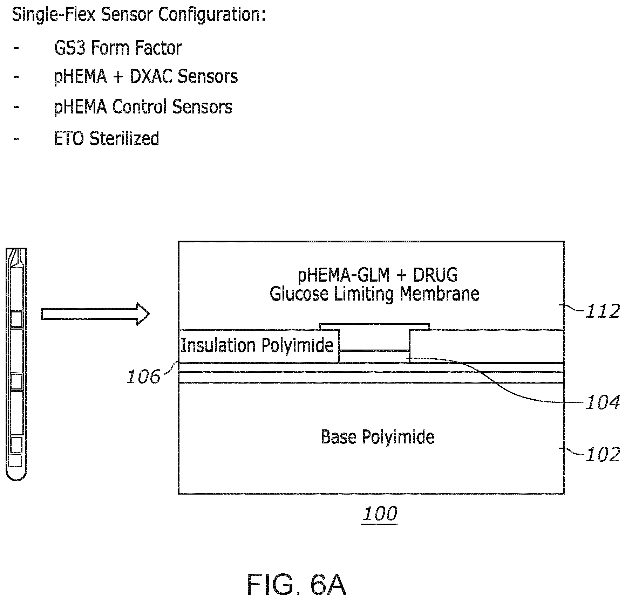

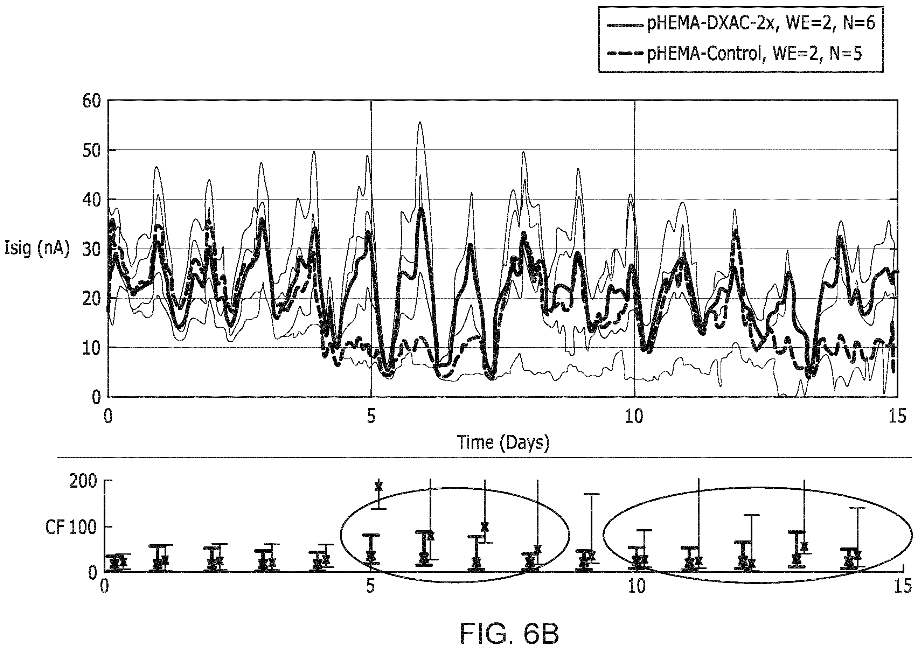

**Figures 6A-6B** provides a cartoon schematic of a layered sensor structure (left panel) and data from a study of sensor stability. Figure 6A provides a schematic of a longitudinal sensor flex element having layered sensor materials disposed thereon (left panel) as well as a cross section of the layered sensor structure disposed on this flex element (right panel). Figure 6B provides data from a study of sensor signals over time (15 days) from sensors coated with a bioactive agent (dexamethasone) as compared to uncoated sensors. The upper panel in Figure 6B shows the raw signal (Isig) data from this 15 day study of these sensor signals; and the lower panel in Figure 6B shows a statistical analysis (cumulative frequency, "CF") of the raw sensor signals/datapoints shown in the upper panel.

### DETAILED DESCRIPTION OF THE EMBODIMENTS

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted. A number of terms are defined below. All publications mentioned herein are incorporated herein by reference to disclose and describe the methods and/or materials in connection with which the publications are cited. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

It must be noted that as used herein and in the appended claims, the singular forms "a", "and", and "the" include plural referents unless the context clearly dictates otherwise. Thus, for example, reference to "an oxidoreductase" includes a plurality of such oxidoreductases and equivalents thereof known to those skilled in the art, and so forth. All numbers recited in the specification and associated claims that refer to values that can be numerically characterized with a value other than a whole number (e.g. "50 mol%") are understood to be modified by the term "about".

The term "analyte" as used herein is a broad term and is used in its ordinary sense, including, without limitation, to refer to a substance or chemical constituent in a fluid such as a biological fluid (for example, blood, interstitial fluid, cerebral spinal fluid, lymph fluid or urine) that can be analyzed. Analytes can include naturally occurring substances, artificial substances, metabolites, and/or reaction products. In some embodiments, the analyte for measurement by the sensing regions, devices, and methods is glucose. However, other analytes are contemplated as well, including but not limited to, lactate. Salts, sugars, proteins fats, vitamins and hormones naturally occurring in blood or interstitial fluids can constitute analytes in certain embodiments. The analyte can be naturally present in the biological fluid or endogenous; for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin. The metabolic products of drugs and pharmaceutical compositions are also contemplated analytes.

The term "sensor," as used herein, is a broad term and is used in its ordinary sense, including, without limitation, the portion or portions of an analyte-monitoring device that detects an analyte. In one embodiment, the sensor includes an electrochemical cell that has a working electrode, a reference electrode, and optionally a counter electrode passing through and secured within the sensor body forming an electrochemically reactive surface at one location on the body, an electronic connection at another location on the body, and a membrane system affixed to the body and covering the electrochemically reactive surface. During general operation of the sensor, a biological sample (for example, blood or interstitial fluid), or a portion thereof, contacts (directly or after passage through one or more membranes or domains) an enzyme (for example, glucose oxidase); the reaction of the biological sample (or portion thereof) results in the formation of reaction products that allow a determination of the analyte level in the biological sample.

As discussed in detail below, embodiments of the invention relate to the use of an electrochemical sensor that exhibits a novel constellation of material and functional elements. Such sensors use bioactive agents disposed within acrylate hydrogel compositions in order to form, for example, analyte sensors having a unique set of technically desirable material properties including increased biocompatibility. The electrochemical sensor embodiments of the invention are designed to measure a concentration of an analyte of interest (e.g. glucose) or a substance indicative of the concentration or presence of the analyte in fluid. In some embodiments, the sensor is a continuous device, for example a subcutaneous, transdermal, or intravascular device. In some embodiments, the device can analyze a plurality of intermittent blood samples. The sensor embodiments disclosed herein can use any known method, including invasive, minimally invasive, and non-invasive sensing techniques, to provide an output signal indicative of the concentration of the analyte of interest. Typically, the sensor is of the type that senses a product or reactant of an enzymatic reaction between an analyte and an enzyme in the presence of oxygen as a measure of the analyte in vivo or in vitro. Such sensors comprise a acrylate hydrogel membrane surrounding the enzyme through which an analyte migrates prior to reacting with the enzyme. The product is then measured using electrochemical methods and thus the output of an electrode system functions as a measure of the analyte.

Embodiments of the invention disclosed herein provide sensors of the type used, for example, in subcutaneous or transcutaneous monitoring of blood glucose levels in a diabetic patient. A variety of implantable, electrochemical biosensors have been developed for the treatment of diabetes and other life-threatening diseases. Many existing sensor designs use some form of immobilized enzyme to achieve their bio-specificity. Embodiments of the invention described herein can be adapted and implemented with a wide variety of known electrochemical sensors, including for example, U.S. Patent Application No. 20050115832, U.S. Pat. Nos. 6,001,067, 6,702,857, 6,212,416, 6,119,028, 6,400,974, 6,595,919, 6,141,573, 6,122,536, 6,512,939 5,605,152, 4,431,004, 4,703,756, 6,514,718, 5,985,129, 5,390,691, 5,391, 250, 5,482,473, 5,299,571, 5,568,806, 5,494,562, 6,120,676, 6,542,765 as well as PCT International Publication Numbers WO 01/58348, WO 04/021877, WO 03/034902, WO 03/035117, WO 03/035891, WO 03/023388, WO 03/022128, WO 03/022352, WO 03/023708, WO 03/036255, WO03/036310 WO 08/042625, and WO 03/074107, and European Patent Application EP 1153571, the contents of each of which are incorporated herein by reference.

As discussed in detail below, embodiments of the invention disclosed herein provide sensor elements having enhanced material properties and/or architectural configurations and sensor systems (e.g. those comprising a sensor and associated electronic components such as a monitor, a processor and the like) constructed to include such elements. The disclosure further provides methods for making and using such sensor membranes and/or architectural configurations. While some embodiments of the invention pertain to glucose sensors, a variety of the elements disclosed herein (e.g. acrylate hydrogel compositions comprising bioactive agents) can be adapted for use with any one of the wide variety of sensors and other implantable medical devices known in the art. The analyte sensor elements, architectures and methods for making and using these elements that are disclosed herein can be used to establish a variety of layered sensor structures.

Specific aspects of embodiments of the invention are discussed in detail in the following sections.

### TYPICAL ELEMENTS, CONFIGURATIONS AND ANALYTE SENSORS OF THE INVENTION

### OPTIMIZED SENSOR ELEMENTS OF THE INVENTION

A wide variety of sensors and sensor elements are known in the art including amperometric sensors used to detect and/or measure biological analytes such as glucose. Many glucose sensors are based on an oxygen (Clark-type) amperometric transducer (see, e.g. Yang et al., Electroanalysis 1997, 9, No. 16: 1252-1256; Clark et al., Ann. N.Y. Acad. Sci. 1962, 102, 29; Updike et al., Nature 1967, 214,986; and Wilkins et al., Med. Engin. Physics, 1996, 18, 273.3-51). A number of *in vivo* glucose sensors utilize hydrogen peroxide-based amperometric transducers because such transducers are relatively easy to fabricate and can readily be miniaturized using conventional technology. One problem associated with the use of certain amperometric transducers, however, include a suboptimal reaction stoichiometry and patient immune responses. As discussed in detail below, these problems are addressed by using the acrylate hydrogel compositions disclosed herein which are designed to release bioactive agents, for example according to a preferred release profile.

In certain sensors such as glucose sensors used by diabetic individuals, the use of bioactive agents such as dexamethasone can improve patient responses to sensor implantation. However, certain glucose oxidase based amperometric glucose sensors comprising bioactive agents can exhibit poor linearity performance of analyte sensing immediately after implantation (e.g. on day 1) in *in-vivo* human studies. Without being bound by a specfic theory or mechanism of action, it is believed that this problem is due to an initial "burst" of bioactive agent that is immediately released into the tissue, a phenomena which can impair the glucose sensing electrode performance. Solutions for this problem that described below include elements and device architectures that are designed to modulate the amount of bioactive agent being release during an early/initial wear period (e.g. the first 12, 24 or 48 hours) as well as elements and device architectures that are designed to limit the amount of bioactive agent that is released near the glucose sensing electrode of the sensor.

One embodiment of the invention is an amperometric analyte sensor comprising a working electrode having a selected constellation of layered elements. Typically, these elements comprise a base layer; a conductive layer disposed over the base layer; an analyte sensing layer disposed over the conductive layer; and an analyte modulating layer disposed over the analyte sensing layer. In such embodiments, the analyte modulating layer is formed from materials that allow this layer to permselectively modulate the diffusion of glucose and oxygen therethrough such that the diffusion of glucose is limited relative to oxygen; and is formed from an acrylate hydrogel having a polymer reversibly coupled to a bioactive agent so that the bioactive agent is uncoupled from the polymer in response to a stimulus. Typically in these embodiments, the analyte modulating layer exhibits a permeability to glucose and oxygen that is altered by less than 10% following release of the bioactive agent from the analyte modulating layer. As shown in Figures 5 and 6, when these acrylate hydrogel membranes are used in amperometric glucose sensors implanted in vivo, the resultant sensors exhibit improved sensor longevity as compared to control sensors having compositions formed from the same materials without the bioactive agents.

In certain embodiments of the invention, the amperometric analyte sensor consists of a single sensor flex assembly comprising a flexible planar element having a longitudinal member comprising a first side and a second side upon which the working electrode is disposed. In embodiments of the invention, the bioactive agent can be mixed within, or alternatively coupled on an external surface of the analyte modulating layer, and comprises at least one of: an antibacterial agent, an anti-inflammatory agent and an anticoagulant agent (e.g., at least one of a dexamethasone, a heparin or a fluoroquinolone). In typical embodiments of the invention, the bioactive agent is noncovalently entrapped within the polymer; and/or the bioactive agent is covalently coupled to the polymer (e.g., where the bioactive agent is coupled to the polymer by an acrylate moiety disposed on the bioactive agent). Typically, the analyte modulating layer comprises at least one of: a Poly(2-hydroxyethyl methacrylate), a polyurethane and a chain extender.

In illustrative embodiments of the invention, the bioactive agent is uncoupled from the polymer within the analyte modulating layer in response to: exposure to aqueous media; and/or exposure to glucose. In some embodiments of the invention, the bioactive agent is uncoupled from the polymer in response to an alteration in the pH of the environment in which the amperometric analyte sensor is disposed. In other embodiments of the invention, the bioactive agent is uncoupled from the polymer in response to an alteration in the temperature of the environment in which the amperometric analyte sensor is disposed. In other embodiments of the invention, the bioactive agent is uncoupled from the polymer in response to an electrochemical stimulus selected from: a voltage applied to the amperometric analyte sensor; and/or a current within the amperometric analyte sensor.

Embodiments of the invention also include methods of making an amperometric analyte sensor electrode for implantation within a mammal. Typically, such methods include the steps of forming a working electrode comprising: a base layer; a conductive layer formed on the base layer; an analyte sensing layer disposed on the conductive layer; and an analyte modulating layer formed on the analyte sensing layer. In such embodiments, the analyte modulating layer is formed from materials selected to permselectively modulate the diffusion of glucose and oxygen therethrough such that the diffusion of glucose is limited relative to oxygen; and the analyte modulating layer is formed to comprise an acrylate hydrogel having a polymer reversibly coupled to a bioactive agent such that the bioactive agent is uncoupled from the polymer in response to a stimuli. In these methods, bioactive agent is typically selected to be at least one of: an antibacterial agent, an anti-inflammatory agent and an anticoagulant agent (e.g., a dexamethasone, a heparin or a fluoroquinolone). In certain embodiments of the invention, the bioactive agent is formed to comprise an acrylate moiety which couples the bioactive agent to the polymer. Typically, the analyte modulating layer comprises at least one of: a Poly(2-hydroxyethyl methacrylate), a polyurethane and a chain extender. In certain embodiments of the invention, the analyte modulating layer is formed from a reaction mixture comprising a photoactive agent selected to facilitate polymerization. Some methods of the invention further comprise performing a sterilization step on the sensor electrode, wherein the sterilization step comprises exposure to ethylene oxide.

In illustrative embodiments of these methods, the bioactive agent is formed to be noncovalently entrapped within the polymer; and/or the bioactive agent is formed to be covalently coupled to the polymer (e.g., where a bioactive agent is coupled to the polymer by an acrylate moiety disposed on the bioactive agent). Optionally, the bioactive agent is coupled to an external surface of the analyte modulating layer. In certain embodiments, the method comprises forming the sensor electrode from materials selected to modulate the hydrophilicity of the analyte modulating layer when the sensor electrode is disposed in an interstitial space. In some methods of the invention, the bioactive agent is formed in the sensor so that it is uncoupled from the polymer in response to an alteration in the pH of the environment in which the amperometric analyte sensor is disposed. In other embodiments of the invention, the bioactive agent is formed in the sensor so that it is uncoupled from the polymer in response to an alteration in the temperature of the environment in which the amperometric analyte sensor is disposed.

In some embodiments of the invention, the bioactive agent is formed in the sensor so that it is uncoupled from the polymer in response to an electrochemical stimuli selected from: a voltage applied to the amperometric analyte sensor; and/or a current within the amperometric analyte sensor. Such embodiments can be adapted to use, for example, electrochemical stimuli disclosed in Szunerits et al., European Polymer Journal 83 (2016) 467-477, the contents of which are incorporated by reference. Szunerits et al., teach that changing the bias of the applied potential, the current density, using continuous or pulsed conditions, negative or positive potential bias, short or long cycles all allow, in a unique manner, the on-demand release of drugs. In other embodiments of the invention, the bioactive agent is formed in the sensor so that it is uncoupled from the polymer in response to endogenous stimuli present in the environment in which the sensor is disclosed (e.g., enzyme, active oxygen species such as hydrogen peroxide, temperature, ions, pH and the like). Such embodiments can be adapted to use, for example, stimuli disclosed in Lin et al., International Journal of Pharmaceutics 602 (2021) 120591, the contents of which are incorporated by reference.

As discussed below, amounts of bioactive agent released from an analyte modulating layer in an analyte sensor can be modulated in a number of different ways. These include, for example, using an analyte sensor designed so that a bioactive agent is either coupled to the exterior surface of the acrylate hydrogel membrane; or alternatively is mixed throughout the acrylate hydrogel membrane and/or by creating a reservoir for the bioactive agent (e.g. a plurality of concentrations of the bioactive agent disposed at the same or different locations within the membrane); and/or differential bioactive agent membrane layer concentrations and/or thicknesses; and/or incorporating bioactive agent in bioabsorbable polymers that release the agent over time; and/or covering the bioactive agent layer with outer bioabsorbable layer that can slow down the drug release; and/or combinations of such elements and device architectures.

In certain embodiments of the invention, the acrylate hydrogel comprises a plurality of sublayers where a least one of the plurality of sublayers comprises a polyurethane composition known in the art (see, e.g. Szycher's Handbook of Polyurethanes 2nd Edition by Michael Szycher Ph.D (Editor)). Such compositions can include, for example, anywhere from 10-90% polyurethane (and 90-10% drug). In embodiments of the invention, the therapeutic release profile of the bioactive agent can be modulated by a number of ways, for example by modifying molecular weights of a acrylate hydrogel materials in which the bioactive is disposed, and/or by using different blending polymers in such compositions, polymers that can be selected to have different glass transition temperatures (Tgs). For example, blending polymers such as Tecoflex SG-60D with other polymers including those disclosed herein can be highly advantageous to tune bioactive agent release characteristics. See, for example, U.S. Patent No. 6,770,729 and U.S. Patent Publication No. 2004/0033251, the contents of which are incorporated herein by reference. Optionally, at least one of the plurality of sublayers is formed by a reaction mixture comprising: a diisocyanate; a hydrophilic polymer comprising a hydrophilic diol or hydrophilic diamine; a siloxane having an amino, hydroxyl or carboxylic acid functional group at a terminus; and optionally a polycarbonate diol.

As discussed in detail below, in typical embodiments of the invention, the acrylate hydrogel material used to make the analyte modulating layer and/or sublayers, the amount of and/or thickness of the sublayers, and the concentration of the bioactive agent in the sublayers is precisely controlled so to create one or more specific release profiles for the bioactive agent. For example, in certain embodiments of the invention, following implantation into the interstitial space of the individual, the plurality of sublayers releases the bioactive agent from the analyte modulating layer according to a bioactive agent profile wherein: not more than 10% of the bioactive agent is released in the first 24 hours after implantation; not more than 20% of the bioactive agent is released in the first 72 hours after implantation; not more than 30% of the bioactive agent is released in the first 120 hours after implantation; and/or at least 30% of the bioactive agent is released in the first 24 hours after implantation; at least 50% of the bioactive agent is released in the first 48 hours after implantation; and/or at least 70% of the bioactive agent is released in the first 72 hours after implantation.

As discussed below, in certain embodiments, the amperometric analyte sensor further includes at least one of: an adhesion promoting layer; a protein layer; a layer comprising poly-l-lysine polymers having molecular weights between 30 KDa and 300KDa; and a cover layer disposed on the analyte sensor apparatus, wherein the cover layer comprises an aperture positioned on the cover layer so as to facilitate an analyte present in an *in vivo* environment from contacting and diffusing through an analyte modulating layer; and contacting the analyte sensing layer.

Embodiments of the invention include methods of making the sensors disclosed herein. For example, embodiments of the invention include a method of making an analyte sensor for implantation within a mammal comprising the steps of: providing a base layer; forming a conductive layer on the base layer, wherein the conductive layer includes a working electrode; forming an analyte sensing layer on the conductive layer, wherein the analyte sensing layer includes an oxidoreductase; and forming an analyte modulating layer on the analyte sensing layer, wherein the analyte modulating layer comprises an bioactive agent selected to inhibit an immune response to the amperometric analyte sensor implanted in an interstitial space of an individual. In typical embodiments of the invention, the analyte modulating layer is further formed to exhibit a first permeability to glucose and a second permeability to O₂, and the permeability to O₂ is greater than the permeability to glucose. In some embodiments of the invention, the amperometric analyte sensor is formed to comprise at least one reservoir in which analyte modulating layer material is disposed.

Typically in these methods, the analyte modulating layer is formed from a plurality of sublayers. For example, in embodiments of the invention, the plurality of sublayers is formed to comprise at least two sublayers selected from the group consisting of: a sublayer comprising a first thickness and/or a first concentration of an bioactive agent; a sublayer comprising a second thickness and/or a second concentration of an bioactive agent; a sublayer comprising a third thickness and/or a third concentration of an bioactive agent; a sublayer comprising a fourth thickness and/or fourth concentration of an bioactive agent; and a sublayer comprising no bioactive agent. In certain embodiments of the invention, at least one of the plurality of sublayers is formed to comprise a polyurethane composition. Optionally, at least one of the plurality of sublayers is formed by a reaction mixture comprising: a diisocyanate; a hydrophilic polymer comprising a hydrophilic diol or hydrophilic diamine; a siloxane having an amino, hydroxyl or carboxylic acid functional group at a terminus; and optionally a polycarbonate diol.

In certain methods of making the analyte sensors of the invention, the sublayers are formed in such methods such that following implantation into the interstitial space of the individual, the plurality of sublayers releases the bioactive agent from the analyte modulating layer according to a profile wherein: not more than 10% of the bioactive agent is released in the first 24 hours after implantation; not more than 20% of the bioactive agent is released in the first 72 hours after implantation; not more than 30% of the bioactive agent is released in the first 120 hours after implantation; and/or at least 30% of the bioactive agent is released in the first 24 hours after implantation; at least 50% of the bioactive agent is released in the first 48 hours after implantation; or at least 70% of the bioactive agent is released in the first 72 hours after implantation.

As discussed below, additional embodiments of the invention include methods of sensing an analyte within the body of a mammal, the methods comprising: implanting an electrochemical analyte sensor disclosed herein in to the mammal; sensing an alteration in current at the working electrode in the presence of the analyte; and then correlating the alteration in current with the presence of the analyte, so that the analyte is sensed.

Additional embodiments of the invention include methods of inhibiting an immune response by using a sensor disclosed herein, thereby extending the life of an implanted device within the body of a mammal. These methods typically comprise implanting an electrochemical analyte sensor disclosed herein (i.e. one comprising an agent that inhibits an immune response at the site of implantation) in to the mammal; sensing an alteration in current at the working electrode in the presence of the analyte; and then correlating the alteration in current with the presence of the analyte, so that the analyte is sensed; wherein the an agent that inhibits an immune response extends the implanted lifespan of the sensor by inhibiting host immune response.

As discussed above, in typical embodiments, an bioactive agent disposed within the analyte modulating layer comprises dexamethasone. However, a wide variety of agents can be used in various embodiments of the invention. For example, the anti-inflammatory agent may be a heparin, rapamycin (sirolimus), tacrolimus, hyaluronidase (e.g. Hylenex^{™}) or combinations thereof. In other embodiments, the anti-inflammatory agent is a methasone (e.g. betamethasone sodium phosphate, dexamethasone sodium phosphate, beclomethasone dipropionate or the like). In yet other embodiments, the anti-inflammatory agent is an anti-inflammatory cytokine or chemokine such as IL-4 or IL-10, or Fractalkine.

Additional examples of anti-inflammatory drugs include both steroidal and non-steroidal (NSAID) anti-inflammatories such as, without limitation, clobetasol, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cortodoxone, deflazacort, desonide, desoximetasone,momentasone, cortisone, cortisone acetate, hydrocortisone, prednisone, prednisone acetate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, tacrolimus and pimecrolimus.

Additionally, examples of steroidal anti-inflammatory drugs include, without limitation, 21-acetoxypregnenolone, alclometasone, algestone, amcinonide, budesonide, chloroprednisone, clobetasol, clobetasone, clocortolone, cloprednol, corticosterone, cortisone, cortivazol, deflazacort, desonide, desoximetasone, diflorasone, diflucortolone, difluprednate, enoxolone, fluazacort, flucloronide, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, any of their derivatives, and combinations thereof.

Furthermore, examples of nonsteroidal anti-inflammatory drugs include, without limitation, COX-1 and COX nonspecific inhibitors (e.g., salicylic acid derivatives, aspirin, sodium salicylate, choline magnesium trisalicylate, salsalate, diflunisal, sulfasalazine and olsalazine; para-aminophenol derivatives such as acetaminophen; indole and indene acetic acids such as indomethacin and sulindac; heteroaryl acetic acids such as tolmetin, dicofenac and ketorolac; arylpropionic acids such as ibuprofen, naproxen, flurbiprofen, ketoprofen, fenoprofen and oxaprozin), and selective COX-2 inhibitors (e.g., diaryl-substituted furanones such as rofecoxib; diaryl-substituted pyrazoles such as celecoxib; indole acetic acids such as etodolac and sulfonanilides such as nimesulide), and combinations thereof. Additionally, other naturally occurring or synthetic drugs, agents, molecules, and proteins may be included with the response-inhibiting agent to mitigate foreign-body responses and/or help facilitate the body in absorbing the medication. For example, Hylenex^{™} (hyaluronidase) may be also included in the delivery path of insulin to increase absorption of the insulin.

As noted above, embodiments of the invention include sensor membranes made from polymeric reaction mixtures formed to include bioactive agents while simultaneously being more permeable to O₂ than to glucose. As is known in the art, a polymer comprises a long or larger molecule consisting of a chain or network of many repeating units, formed by chemically bonding together many identical or similar small molecules called monomers. A copolymer or heteropolymer is a polymer derived from two (or more) monomeric species, as opposed to a homopolymer where only one monomer is used. Copolymers may also be described in terms of the existence of or arrangement of branches in the polymer structure. Linear copolymers consist of a single main chain whereas branched copolymers consist of a single main chain with one or more polymeric side chains. Sensor membranes made from polymeric compositions comprising bioactive agents disclosed herein can optimize analyte sensor function including biocompatibility, sensor sensitivity, stability and hydration profiles. In addition, by optimizing the stoichiometry of reactant species over a range of sensor temperatures, the membranes disclosed herein can optimize the chemical reactions that produce the critical measurable signals that correlate with the levels of an analyte of interest (e.g. glucose). The following sections describe illustrative sensor elements, sensor configurations and methodological embodiments of the invention.

Another embodiment of the invention is an amperometric analyte sensor comprising a base layer, a conductive layer disposed on the base layer and comprising a working electrode, an analyte sensing layer disposed on the conductive layer, and an analyte modulating layer comprising an bioactive agent disposed on the analyte sensing layer. In this embodiment, the analyte modulating layer is formed by a reaction mixture comprising a diisocyanate, a hydrophilic polymer comprising a hydrophilic diol or hydrophilic diamine, a siloxane having an amino, hydroxyl or carboxylic acid functional group at a terminus, and a catalyst. In certain embodiments, the amount of catalyst present in the reaction mixture in amounts less than 0.2% of reaction mixture components so that the analyte modulating layer exhibits a greater thermal stability than a comparable analyte modulating layer formed from a reaction mixture where the catalyst is present in the formulation in amounts greater than or equal to 0.2% of the reaction mixture.

In typical embodiments, the analyte sensor is a glucose sensor that is implantable *in vivo.* Optionally, the analyte sensor further comprises at least one of: a protein layer disposed on the analyte sensing layer, or a cover layer disposed on the analyte sensor apparatus, and the cover layer comprises an aperture positioned on the cover layer so as to facilitate an analyte present in an *in vivo* environment from contacting and diffusing through an analyte modulating layer; and contacting the analyte sensing layer. In certain of these analyte sensors, the conductive layer comprises a plurality of electrodes including a working electrode, a counter electrode and a reference electrode, for example an embodiment where the conductive layer comprises a plurality of working electrodes and/or counter electrodes and/or reference electrodes; and optionally the plurality of working, counter and reference electrodes are grouped together as a unit and positionally distributed on the conductive layer in a repeating pattern of units.

In embodiments of the invention, the analyte modulating layer is formed by a reaction mixture comprising an acrylate, a diisocyanate, a hydrophilic polymer comprising a hydrophilic diol or hydrophilic diamine, a siloxane having an amino, hydroxyl or carboxylic acid functional group at a terminus; and a catalyst. Optionally the reaction mixture further comprises additional components such as an bioactive agent. In certain methods of making an analyte sensor for implantation within a mammal, the diisocyanate comprises a hexamethylene diisocyanate and/or a methylene diphenyl diisocyanate, the JEFFAMINE comprises about 45% JEFFAMINE 600 and/or JEFFAMINE 900, the polydimethylsiloxane comprises about 22.5% polydimethylsiloxane-A15), and the polycarbonate diol comprises about 7.5% (poly(1,6-hexyle carbonate) diol. Typically in this embodiment, the catalyst (e.g. Dibutyltin bis(2-ethylhexanoate)) is present in the reaction mixture in amounts less than 0.19%, 0.17%, 0.15%, 0.13%, or 0.11% of the reaction mixture (e.g. about 0.1%).

Certain amperometric sensor design used with embodiments of the invention comprise a plurality of layered elements including for example a base layer having an electrode, an analyte sensing layer (e.g. one comprising glucose oxidase) and an analyte modulating layer that functions to both release an bioactive agent as well as in analyte diffusion control (e.g. to modulate the amounts of glucose and oxygen exposed to the analyte sensing layer). One such sensor embodiment is shown in FIG. 2A. Layered sensor designs that incorporate the acrylate hydrogel compositions comprising bioactive agents disclosed herein as the analyte modulating layer exhibit a constellation of material properties that overcome challenges observed in a variety of sensors including electrochemical glucose sensors that are implanted in vivo. For example, sensors designed to measure analytes in aqueous environments (e.g. those implanted *in vivo*) typically require wetting of the layers prior to and during the measurement of accurate analyte reading. Because the properties of a material can influence the rate at which it hydrates, the material properties of membranes used in aqueous environments ideally will facilitate sensor wetting to, for example, minimize the time period between the sensor's introduction into an aqueous environment and its ability to provide accurate signals that correspond to the concentrations of an analyte in that environment. Embodiments of the invention that comprise acrylate hydrogel compositions comprising bioactive agents address such issues by facilitating sensor hydration and biocompatibility simultaneously.

Moreover, with electrochemical glucose sensors that utilize the chemical reaction between glucose and glucose oxidase to generate a measurable signal, the material of the analyte modulating layer should not exacerbate (and ideally should diminish) what is known in the art as the "oxygen deficit problem". Specifically, because glucose oxidase-based sensors require both oxygen (O₂) as well as glucose to generate a signal, the presence of an excess of oxygen relative to glucose, is necessary for the operation of a glucose oxidase-based glucose sensor. However, because the concentration of oxygen in subcutaneous tissue is much less than that of glucose, oxygen can be the limiting reactant in the reaction between glucose, oxygen, and glucose oxidase in a sensor, a situation which compromises the sensor's ability to produce a signal that is strictly dependent on the concentration of glucose. In this context, because the properties of a material can influence the rate at which compounds diffuse through that material to the site of a measurable chemical reaction, the material properties of an analyte modulating layer used in electrochemical glucose sensors that utilize the chemical reaction between glucose and glucose oxidase to generate a measurable signal, should not for example, favor the diffusion of glucose over oxygen in a manner that contributes to the oxygen deficit problem. Embodiments of the invention that comprise the acrylate hydrogel compositions comprising bioactive agents disclosed herein do not contribute to, and instead function to ameliorate, the oxygen deficit problem. Typically for example, the analyte modulating layer is formed to exhibit a first permeability to glucose and a second permeability to O₂, and the permeability to O₂ is greater than the permeability to glucose.

Embodiments of the invention include both materials (e.g. acrylate hydrogel compositions comprising bioactive agents) as well as architectures that designed to facilitate sensor performance. For example, in certain embodiments of the invention, the conductive layer is formed on a flexible sensor base (e.g. a sensor flex assembly) that comprises a plurality of working electrodes and/or counter electrodes and/or reference electrodes (e.g. 3 working electrodes, a reference electrode and a counter electrode), in order to, for example, avoid problems associated with poor sensor hydration and/or provide redundant sensing capabilities. Optionally, the plurality of working, counter and reference electrodes are configured together as a unit and positionally distributed on the conductive layer in a repeating pattern of units. In certain embodiments of the invention, the base layer is made from a flexible material that allows the sensor to twist and bend when implanted in vivo; and the electrodes are grouped in a configuration that facilitates an in vivo fluid contacting at least one of working electrode as the sensor apparatus twists and bends when implanted in vivo. In some embodiments, the electrodes are grouped in a configuration that allows the sensor to continue to function if a portion of the sensor having one or more electrodes is dislodged from an in vivo environment and exposed to an ex vivo environment. Typically, the sensor is operatively coupled to a sensor input capable of receiving a signal from the sensor that is based on a sensed analyte; and a processor coupled to the sensor input, wherein the processor is capable of characterizing one or more signals received from the sensor. In some embodiments of the invention, a pulsed voltage is used to obtain a signal from one or more electrodes of a sensor.

The sensors disclosed herein can be made from a wide variety of materials known in the art. In one illustrative embodiment of the invention, the analyte modulating layer comprises a Poly(2-hydroxyethyl methacrylate) and polyurethane/polyurea polymer that is typically formed from a mixture comprising: a polymerizable acrylate, a diisocyanate; a hydrophilic polymer comprising a hydrophilic diol or hydrophilic diamine; and a siloxane having an amino, hydroxyl or carboxylic acid functional group at a terminus; with this polymer then polycarbonate with a branched acrylate polymer formed from a mixture comprising: a butyl, propyl, ethyl or methyl-acrylate; an amino-acrylate; a siloxane-acrylate; and a polyethylene oxide)-acrylate. Optionally, additional materials can be included in these polymeric blends. For example, certain embodiments of the branched acrylate polymer are formed from a reaction mixture that includes a hydroxyl-acrylate compound (e.g. 2-hydroxyethyl methacrylate).

As used herein, the term "polyurethane/polyurea polymer" refers to a polymer containing urethane linkages, urea linkages or combinations thereof. As is known in the art, polyurethane is a polymer consisting of a chain of organic units joined by urethane (carbamate) links. Polyurethane polymers are typically formed through step-growth polymerization by reacting a monomer containing at least two isocyanate functional groups with another monomer containing at least two hydroxyl (alcohol) groups in the presence of a catalyst. Polyurea polymers are derived from the reaction product of an isocyanate component and a diamine. Typically, such polymers are formed by combining diisocyanates with alcohols and/or amines. For example, combining isophorone diisocyanate with PEG 600 and aminopropyl polysiloxane under polymerizing conditions provides a polyurethane/polyurea composition having both urethane (carbamate) linkages and urea linkages. Such polymers are well known in the art and described for example in U.S. Patent Nos. 5,777,060, 5,882,494 and 6,632,015, and PCT publications WO 96/30431; WO 96/18115; WO 98/13685; and WO 98/17995, the contents of each of which is incorporated by reference.

The polyurethane/polyurea compositions of the invention are prepared from biologically acceptable polymers whose hydrophobic/hydrophilic balance can be varied over a wide range to control the ratio of the diffusion coefficient of oxygen to that of glucose, and to match this ratio to the design requirements of electrochemical glucose sensors intended for in vivo use. Such compositions can be prepared by conventional methods by the polymerization of monomers and polymers noted above. The resulting polymers are soluble in solvents such as acetone or ethanol and may be formed as a membrane from solution by dip, spray or spin coating.

Diisocyanates useful in this embodiment of the invention are those which are typically those which are used in the preparation of biocompatible polyurethanes. Such diisocyanates are described in detail in Szycher, SEMINAR ON ADVANCES IN MEDICAL GRADE POLYURETHANES, Technomic Publishing, (1995) and include both aromatic and aliphatic diisocyanates. Examples of suitable aromatic diisocyanates include toluene diisocyanate, 4,4'-diphenylmethane diisocyanate, 3,3'-dimethyl-4,4'-biphenyl diisocyanate, naphthalene diisocyanate and paraphenylene diisocyanate. Suitable aliphatic diisocyanates include, for example, 1,6hexamethylene diisocyanate (HDI), trimethylhexamethylene diisocyanate (TMDI), trans1,4-cyclohexane diisocyanate (CHDI), 1,4-cyclohexane bis(methylene isocyanate) (BDI), 1,3-cyclohexane bis(methylene isocyanate) (H₆ XDI), isophorone diisocyanate (IPDI) and 4,4'-methylenebis(cyclohexyl isocyanate) (H₂ MDI). In some embodiments, the diisocyanate is isophorone diisocyanate, 1,6-hexamethylene diisocyanate, or 4,4'methylenebis(cyclohexyl isocyanate). A number of these diisocyanates are available from commercial sources such as Aldrich Chemical Company (Milwaukee, Wis., USA) or can be readily prepared by standard synthetic methods using literature procedures.

The quantity of diisocyanate used in the reaction mixture for the polyurethane/polyurea polymer compositions is typically about 50 mol % relative to the combination of the remaining reactants. More particularly, the quantity of diisocyanate employed in the preparation of the polyurethane/polyurea polymer will be sufficient to provide at least about 100% of the --NCO groups necessary to react with the hydroxyl or amino groups of the remaining reactants. For example, a polymer which is prepared using x moles of diisocyanate, will use a moles of a hydrophilic polymer (diol, diamine or combination), b moles of a silicone polymer having functionalized termini, and c moles of a chain extender, such that x=a+b+c, with the understanding that c can be zero.

Another reactant used in the preparation of the polyurethane/polyurea polymers described herein is a hydrophilic polymer. The hydrophilic polymer can be a hydrophilic diol, a hydrophilic diamine or a combination thereof. The hydrophilic diol can be a poly(alkylene)glycol, a polyester-based polyol, or a polycarbonate polyol. As used herein, the term "poly(alkylene)glycol" refers to polymers of lower alkylene glycols such as poly(ethylene)glycol, poly(propylene)glycol and polytetramethylene ether glycol (PTMEG). The term "polyester-based polyol" refers to a polymer in which the R group is a lower alkylene group such as ethylene, 1,3-propylene, 1,2-propylene, 1,4-butylene,2,2-dimethyl-1,3-propylene, and the like (e.g. as depicted in FIG. 4 of U.S. Patent Nos. 5,777,060). One of skill in the art will also understand that the diester portion of the polymer can also vary from the six-carbon diacid shown. For example, while FIG. 4 of U.S. Patent Nos. 5,777,060 illustrates an adipic acid component, the present invention also contemplates the use of succinic acid esters, glutaric acid esters and the like. The term "polycarbonate polyol" refers those polymers having hydroxyl functionality at the chain termini and ether and carbonate functionality within the polymer chain. The alkyl portion of the polymer will typically be composed of C2 to C4 aliphatic radicals, or in some embodiments, longer chain aliphatic radicals, cycloaliphatic radicals or aromatic radicals. The term "hydrophilic diamines" refers to any of the above hydrophilic diols in which the terminal hydroxyl groups have been replaced by reactive amine groups or in which the terminal hydroxyl groups have been derivatized to produce an extended chain having terminal amine groups. For example, some hydrophilic diamines are a "diamino poly(oxyalkylene)" which is poly(alkylene)glycol in which the terminal hydroxyl groups are replaced with amino groups. The term "diamino poly(oxyalkylene" also refers to poly(alkylene)glycols which have aminoalkyl ether groups at the chain termini. One example of a suitable diamino poly(oxyalkylene) is polypropylene glycol)bis(2-aminopropyl ether). A number of the above polymers can be obtained from Aldrich Chemical Company. Alternatively, conventional methods known in the art can be employed for their synthesis.

The amount of hydrophilic polymer which is used to make the linear polymer compositions will typically be about 10% to about 80% by mole relative to the diisocyanate which is used. Typically, the amount is from about 20% to about 60% by mole relative to the diisocyanate. When lower amounts of hydrophilic polymer are used, it is common to include a chain extender.

Silicone containing polyurethane/polyurea polymers which are useful in the present invention are typically linear, have excellent oxygen permeability and lower glucose permeability. Typically, the silicone polymer is a polydimethylsiloxane having two reactive functional groups (i.e., a functionality of 2). The functional groups can be, for example, hydroxyl groups, amino groups or carboxylic acid groups, but are typically hydroxyl or amino groups. In some embodiments, combinations of silicone polymers can be used in which a first portion comprises hydroxyl groups and a second portion comprises amino groups. Typically, the functional groups are positioned at the chain termini of the silicone polymer. A number of suitable silicone polymers are commercially available from such sources as Dow Chemical Company (Midland, Mich., USA) and General Electric Company (Silicones Division, Schenectady, N.Y., USA). Still others can be prepared by general synthetic methods known in the art (see, e.g. U.S. Patent Nos. 5,777,060), beginning with commercially available siloxanes (United Chemical Technologies, Bristol. Pa., USA). For use in the present invention, the silicone polymers will typically be those having a molecular weight of from about 400 to about 10,000, more typically those having a molecular weight of from about 2000 to about 4000. The amount of silicone polymer which is incorporated into the reaction mixture will depend on the desired characteristics of the resulting polymer from which the biocompatible membrane is formed. For those compositions in which a lower glucose penetration is desired, a larger amount of silicone polymer can be employed. Alternatively, for compositions in which a higher glucose penetration is desired, smaller amounts of silicone polymer can be employed. Typically, for a glucose sensor, the amount of siloxane polymer will be from 10% to 90% by mole relative to the diisocyanate. Typically, the amount is from about 20% to 60% by mole relative to the diisocyanate.

In one group of embodiments, the reaction mixture for the preparation of biocompatible membranes will also contain a chain extender which is an aliphatic or aromatic diol, an aliphatic or aromatic diamine, alkanolamine, or combinations thereof (e.g. as depicted in FIG. 8 of U.S. Patent Nos. 5,777,060)). Examples of suitable aliphatic chain extenders include ethylene glycol, propylene glycol, 1,4-butanediol, 1,6-hexanediol, ethanolamine, ethylene diamine, butane diamine, 1,4-cyclohexanedimethanol. Aromatic chain extenders include, for example, para-di(2-hydroxyethoxy)benzene, meta-di(2-hydroxyethoxy)benzene, Ethacure 100^{®} (a mixture of two isomers of 2,4-diamino-3,5-diethyltoluene), Ethacure 300^{®} (2,4-diamino-3,5-di(methylthio)toluene), 3,3'-dichloro-4,4'diaminodiphenylmethane, Polacure^{®} 740M (trimethylene glycol bis(para-aminobenzoate)ester), and methylenedianiline. Incorporation of one or more of the above chain extenders typically provides the resulting biocompatible membrane with additional physical strength, but does not substantially increase the glucose permeability of the polymer. Typically, a chain extender is used when lower (i.e., 10-40 mol %) amounts of hydrophilic polymers are used. In particularly some compositions, the chain extender is diethylene glycol which is present in from about 40% to 60% by mole relative to the diisocyanate.

Oxygen and glucose diffusion coefficients can also be determined for the acrylate hydrogel compositions comprising bioactive agents of the present invention. Methods for determining diffusion coefficients are known to those of skill in the art, and examples are provided below. Certain embodiments of the biocompatible membranes described herein will typically have an oxygen diffusion coefficient (D_{oxygen}) of about 0.1 × 10⁻⁶ cm² /sec to about 2.0 × 10⁻⁶ cm² /sec and a glucose diffusion coefficient (D_{glucose}) of about 1 × 10⁻⁹ cm² /sec to about 500 × 10⁻⁹ cm² /sec. More typically, the glucose diffusion coefficient is about 10 × 10⁻⁹ cm² /sec to about 200 × 10⁻⁹ cm² /sec.

### DIAGRAMMATIC ILLUSTRATION OF TYPICAL SENSOR CONFIGURATIONS

Figure 2A illustrates a cross-section of a conventional sensor embodiment 100. The components of the sensor are typically characterized herein as layers in this layered electrochemical sensor stack because, for example, it allows for a facile characterization of conventional sensor structures such as those shown in Figure 2A and their differences from the invention disclosed herein as shown in Figure 2B (i.e. ones comprising a high density amine (HDA) layer comprising poly-l-lysine polymers having molecular weights between 30 KDa and 300KDa). Artisans will understand, that in certain embodiments of the invention, the sensor constituents are combined such that multiple constituents form one or more heterogeneous layers. In this context, those of skill in the art understand that, while certain layers/components of conventional sensor embodiments are useful in the HDA sensors disclosed herein, the placement and composition of the layered constituents is very different in HDA sensor embodiments of the invention. Those of skill in this art will understand that certain embodiments if the invention include elements/layers that are found in conventional sensors while others are excluded, and/or new material layers/elements are included. For example, certain elements disclosed in Figure 2A are also found in the invention disclosed herein (e.g. a base, analyte sensing layer, an analyte modulating layer etc.) while, as shown in Figure 2B, other elements are not (e.g. separate HSA protein layers, layers comprising a siloxane adhesion promoter etc.). Similarly, embodiments of the invention include layers/elements having materials disposed in unique configurations that are not found in conventional sensors (e.g. high-density amine (HDA) polymer layers).

The embodiment shown in FIG. 2A includes a base layer 102 to support the sensor 100. The base layer 102 can be made of a material such as a metal and/or a ceramic and/or a polymeric substrate, which may be self-supporting or further supported by another material as is known in the art. Embodiments of the invention include a conductive layer 104 which is disposed on and/or combined with the base layer 102. Typically the conductive layer 104 comprises one or more electrodes. An operating sensor 100 typically includes a plurality of electrodes such as a working electrode, a counter electrode and a reference electrode. Other embodiments may also include a plurality of working and/or counter and/or reference electrodes and/or one or more electrodes that performs multiple functions, for example one that functions as both as a reference and a counter electrode.

As discussed in detail below, the base layer 102 and/or conductive layer 104 can be generated using many known techniques and materials. In certain embodiments of the invention, the electrical circuit of the sensor is defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. Additional electrically insulating elements such as an insulation polyimide 106 (or cover layer) can be disposed on portions of the sensor 100. Acceptable polymer coatings for use as the electrically insulating element(s) 106 can include, but are not limited to, non-toxic biocompatible polymers such as silicone compounds, polyimides, biocompatible solder masks, epoxy acrylate copolymers, or the like. In the sensors of the present invention, one or more exposed regions or apertures 108 can be made through a cover layer to open the conductive layer 104 to the external environment and to, for example, allow an analyte such as glucose to permeate the layers of the sensor and be sensed by the sensing elements. Apertures 108 can be formed by a number of techniques, including laser ablation, tape masking, chemical milling or etching or photolithographic development or the like. In certain embodiments of the invention, during manufacture, a secondary photoresist can also be applied to the electrically insulating element(s) to define the regions of the protective layer to be removed to form the aperture(s) 108. The exposed electrodes and/or contact pads can also undergo secondary processing (e.g. through the apertures 108), such as additional plating processing, to prepare the surfaces and/or strengthen the conductive regions.

In the sensor configuration shown in FIG. 2A, an analyte sensing layer 110 (which is typically a sensor chemistry layer, meaning that materials in this layer undergo a chemical reaction to produce a signal that can be sensed by the conductive layer) is disposed on one or more of the exposed electrodes of the conductive layer 104. In the sensor configuration shown in Figure 2B, an interference rejection membrane 120 is disposed on one or more of the exposed electrodes of the conductive layer 104, with the analyte sensing layer 110 then being disposed on this interference rejection membrane 120. Typically, the analyte sensing layer 110 is an enzyme layer. Most typically, the analyte sensing layer 110 comprises an enzyme capable of producing and/or utilizing oxygen and/or hydrogen peroxide, for example the enzyme glucose oxidase. Optionally the enzyme in the analyte sensing layer is combined with a second carrier protein such as human serum albumin, bovine serum albumin or the like. In an illustrative embodiment, an oxidoreductase enzyme such as glucose oxidase in the analyte sensing layer 110 reacts with glucose to produce hydrogen peroxide, a compound which then modulates a current at an electrode. As this modulation of current depends on the concentration of hydrogen peroxide, and the concentration of hydrogen peroxide correlates to the concentration of glucose, the concentration of glucose can be determined by monitoring this modulation in the current. In a specific embodiment of the invention, the hydrogen peroxide is oxidized at a working electrode which is an anode (also termed herein the anodic working electrode), with the resulting current being proportional to the hydrogen peroxide concentration. Such modulations in the current caused by changing hydrogen peroxide concentrations can by monitored by any one of a variety of sensor detector apparatuses such as a universal sensor amperometric biosensor detector or one of the other variety of similar devices known in the art such as glucose monitoring devices produced by Medtronic MiniMed.

In embodiments of the invention, the analyte sensing layer 110 can be applied over portions of the conductive layer or over the entire region of the conductive layer. Typically the analyte sensing layer 110 is disposed on the working electrode which can be the anode or the cathode. Optionally, the analyte sensing layer 110 is also disposed on a counter and/or reference electrode. While the analyte sensing layer 110 can be up to about 1000 microns (µm) in thickness, typically the analyte sensing layer or sublayer is relatively thin as compared to those found in sensors previously described in the art, and is for example, typically less than 1, 0.5, 0.25 or 0.1 microns in thickness. As discussed in detail below, some methods for generating a thin analyte sensing layer 110 include brushing the layer onto a substrate (e.g. the reactive surface of a platinum black electrode), as well as spin coating processes, dip and dry processes, low shear spraying processes, ink-jet printing processes, silk screen processes and the like.

Typically, the analyte sensing layer 110 is coated and or disposed next to one or more additional layers. Optionally, the one or more additional layers includes a protein layer 116 disposed upon the analyte sensing layer 110. Typically, the protein layer 116 comprises a protein such as human serum albumin, bovine serum albumin or the like. Typically, the protein layer 116 comprises human serum albumin. In some embodiments of the invention, an additional layer includes an analyte modulating layer 112 that is disposed above the analyte sensing layer 110 to regulate analyte access with the analyte sensing layer 110. For example, the analyte modulating membrane layer 112 can comprise a glucose limiting membrane, which regulates the amount of glucose that contacts an enzyme such as glucose oxidase that is present in the analyte sensing layer. Such glucose limiting membranes can be made from a wide variety of materials known to be suitable for such purposes, e.g., silicone compounds such as polydimethyl siloxanes, polyurethanes, polyurea cellulose acetates, NAFION, polyester sulfonic acids (e.g. Kodak AQ), hydrogels, the polymer blends disclosed herein or any other suitable hydrophilic membranes known to those skilled in the art.

In some embodiments of the invention, an adhesion promoter layer 114 is disposed between layers such as the analyte modulating layer 112 and the analyte sensing layer 110 as shown in FIG. 2A in order to facilitate their contact and/or adhesion. In a specific embodiment of the invention, an adhesion promoter layer 114 is disposed between the analyte modulating layer 112 and the protein layer 116 as shown in FIG. 2A in order to facilitate their contact and/or adhesion. The adhesion promoter layer 114 can be made from any one of a wide variety of materials known in the art to facilitate the bonding between such layers. Typically, the adhesion promoter layer 114 comprises a silane compound. In alternative embodiments, protein or like molecules in the analyte sensing layer 110 can be sufficiently crosslinked or otherwise prepared to allow the analyte modulating membrane layer 112 to be disposed in direct contact with the analyte sensing layer 110 in the absence of an adhesion promoter layer 114.

Embodiments of typical elements used to make the sensors disclosed herein are discussed below.

### TYPICAL ANALYTE SENSOR CONSTITUENTS USED IN EMBODIMENTS OF THE INVENTION

The following disclosure provides examples of typical elements/constituents used in sensor embodiments of the invention. While these elements can be described as discreet units (e.g. layers), those of skill in the art understand that sensors can be designed to contain elements having a combination of some or all of the material properties and/or functions of the elements/constituents discussed below (e.g. an element that serves both as a supporting base constituent and/or a conductive constituent and/or a matrix for the analyte sensing constituent and which further functions as an electrode in the sensor). Those in the art understand that these thin film analyte sensors can be adapted for use in a number of sensor systems such as those described below.

### BASE CONSTITUENT

Sensors of the invention typically include a base constituent (see, e.g. element 102 in FIG. 2A). The term "base constituent" is used herein according to art accepted terminology and refers to the constituent in the apparatus that typically provides a supporting matrix for the plurality of constituents that are stacked on top of one another and comprise the functioning sensor. In one form, the base constituent comprises a thin film sheet of insulative (e.g. electrically insulative and/or water impermeable) material. This base constituent can be made of a wide variety of materials having desirable qualities such as dielectric properties, water impermeability and hermeticity. Some materials include metallic, and/or ceramic and/or polymeric substrates or the like.

The base constituent may be self-supporting or further supported by another material as is known in the art. In one embodiment of the sensor configuration shown in FIG. 2A, the base constituent 102 comprises a ceramic. Alternatively, the base constituent comprises a polymeric material such as a polyimmide. In an illustrative embodiment, the ceramic base comprises a composition that is predominantly Al₂O₃ (e.g. 96%). The use of alumina as an insulating base constituent for use with implantable devices is disclosed in U.S. Pat. Nos. 4,940,858, 4,678,868 and 6,472,122 which are incorporated herein by reference. The base constituents of the invention can further include other elements known in the art, for example hermetical vias (see, e.g. WO 03/023388). Depending upon the specific sensor design, the base constituent can be relatively thick constituent (e.g. thicker than 50, 100, 200, 300, 400, 500 or 1000 microns). Alternatively, one can utilize a nonconductive ceramic, such as alumina, in thin constituents, e.g., less than about 30 microns.

### CONDUCTIVE CONSTITUENT

The electrochemical sensors of the invention typically include a conductive constituent disposed upon the base constituent that includes at least one electrode for measuring an analyte or its byproduct (e.g. oxygen and/or hydrogen peroxide) to be assayed (see, e.g. element 104 in FIG. 2A). The term "conductive constituent" is used herein according to art accepted terminology and refers to electrically conductive sensor elements such as electrodes which are capable of measuring and a detectable signal and conducting this to a detection apparatus. An illustrative example of this is a conductive constituent that can measure an increase or decrease in current in response to exposure to a stimuli such as the change in the concentration of an analyte or its byproduct as compared to a reference electrode that does not experience the change in the concentration of the analyte, a coreactant (e.g. oxygen) used when the analyte interacts with a composition (e.g. the enzyme glucose oxidase) present in analyte sensing constituent 110 or a reaction product of this interaction (e.g. hydrogen peroxide). Illustrative examples of such elements include electrodes which are capable of producing variable detectable signals in the presence of variable concentrations of molecules such as hydrogen peroxide or oxygen. Typically one of these electrodes in the conductive constituent is a working electrode, which can be made from non-corroding metal or carbon. A carbon working electrode may be vitreous or graphitic and can be made from a solid or a paste. A metallic working electrode may be made from platinum group metals, including palladium or gold, or a non-corroding metallically conducting oxide, such as ruthenium dioxide. Alternatively, the electrode may comprise a silver/silver chloride electrode composition. The working electrode may be a wire or a thin conducting film applied to a substrate, for example, by coating or printing. Typically, only a portion of the surface of the metallic or carbon conductor is in electrolytic contact with the analyte-containing solution. This portion is called the working surface of the electrode. The remaining surface of the electrode is typically isolated from the solution by an electrically insulating constituent 106. Examples of useful materials for generating this electrically insulating constituent include polymers such as polyimides, polytetrafluoroethylene, polyhexafluoropropylene and silicones such as polysiloxanes.

In addition to the working electrode, the analyte sensors of the invention typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors of the present invention have one or more working electrodes and one or more counter, reference, and/or counter/reference electrodes. One embodiment of the sensor of the present invention has two, three or four or more working electrodes. These working electrodes in the sensor may be integrally connected or they may be kept separate.

Typically for in vivo use, embodiments of the present invention are implanted subcutaneously in the skin of a mammal for direct contact with the body fluids of the mammal, such as blood. Alternatively, the sensors can be implanted into other regions within the body of a mammal such as in the intraperotineal space. When multiple working electrodes are used, they may be implanted together or at different positions in the body. The counter, reference, and/or counter/reference electrodes may also be implanted either proximate to the working electrode(s) or at other positions within the body of the mammal. Embodiments of the invention include sensors comprising electrodes constructed from nanostructured materials. As used herein, a "nanostructured material" is an object manufactured to have at least one dimension smaller than 100 nm. Examples include, but are not limited to, single-walled nanotubes, double-walled nanotubes, multi-walled nanotubes, bundles of nanotubes, fullerenes, cocoons, nanowires, nanofibres, onions and the like.

### INTERFERENCE REJECTION CONSTITUENT

The electrochemical sensors of the invention optionally include an interference rejection constituent disposed between the surface of the electrode and the environment to be assayed. In particular, certain sensor embodiments rely on the oxidation and/or reduction of hydrogen peroxide generated by enzymatic reactions on the surface of a working electrode at a constant potential applied. Because amperometric detection based on direct oxidation of hydrogen peroxide requires a relatively high oxidation potential, sensors employing this detection scheme may suffer interference from oxidizable species that are present in biological fluids such as ascorbic acid, uric acid and acetaminophen. In this context, the term "interference rejection constituent" is used herein according to art accepted terminology and refers to a coating or membrane in the sensor that functions to inhibit spurious signals generated by such oxidizable species which interfere with the detection of the signal generated by the analyte to be sensed. Certain interference rejection constituents' function via size exclusion (e.g. by excluding interfering species of a specific size). Examples of interference rejection constituents include one or more layers or coatings of compounds such as hydrophilic crosslinked pHEMA and polylysine polymers as well as cellulose acetate (including cellulose acetate incorporating agents such as polyethylene glycol)), polyethersulfones, polytetra-fluoroethylenes, the perfluoronated ionomer NAFION, polyphenylenediamine, epoxy and the like. Illustrative discussions of such interference rejection constituents are found for example in Ward et al., Biosensors and Bioelectronics 17 (2002) 181-189 and Choi et al., Analytical Chimica Acta 461 (2002) 251-260 which are incorporated herein by reference. Other interference rejection constituents include for example those observed to limit the movement of compounds based upon a molecular weight range, for example cellulose acetate as disclosed for example in U.S. Patent No. 5,755,939, the contents of which are incorporated by reference. Additional compositions having an unexpected constellation of material properties that make them ideal for use as interference rejection membranes in certain amperometric glucose sensors as well as methods for making and using them are disclosed herein, for example in U.S. Patent Application Serial Number 12/572,087.

### ANALYTE SENSING CONSTITUENT

The electrochemical sensors of the invention include an analyte sensing constituent disposed on the electrodes of the sensor (see, e.g. element 110 in FIG. 2A). The term "analyte sensing constituent" is used herein according to art accepted terminology and refers to a constituent comprising a material that is capable of recognizing or reacting with an analyte whose presence is to be detected by the analyte sensor apparatus. Typically this material in the analyte sensing constituent produces a detectable signal after interacting with the analyte to be sensed, typically via the electrodes of the conductive constituent. In this regard the analyte sensing constituent and the electrodes of the conductive constituent work in combination to produce the electrical signal that is read by an apparatus associated with the analyte sensor. Typically, the analyte sensing constituent comprises an oxidoreductase enzyme capable of reacting with and/or producing a molecule whose change in concentration can be measured by measuring the change in the current at an electrode of the conductive constituent (e.g. oxygen and/or hydrogen peroxide), for example the enzyme glucose oxidase. An enzyme capable of producing a molecule such as hydrogen peroxide can be disposed on the electrodes according to a number of processes known in the art. The analyte sensing constituent can coat all or a portion of the various electrodes of the sensor. In this context, the analyte sensing constituent may coat the electrodes to an equivalent degree. Alternatively, the analyte sensing constituent may coat different electrodes to different degrees, with for example the coated surface of the working electrode being larger than the coated surface of the counter and/or reference electrode.

Typical sensor embodiments of this element of the invention utilize an enzyme (e.g. glucose oxidase) that has been combined with a second protein (e.g. albumin) in a fixed ratio (e.g. one that is typically optimized for glucose oxidase stabilizing properties) and then applied on the surface of an electrode to form a thin enzyme constituent. In a typical embodiment, the analyte sensing constituent comprises a GOx and HSA mixture. In a typical embodiment of an analyte sensing constituent having GOx, the GOx reacts with glucose present in the sensing environment (e.g. the body of a mammal) and generates hydrogen peroxide, wherein the hydrogen peroxide so generated is anodically detected at the working electrode in the conductive constituent.

As noted above, the enzyme and the second protein (e.g. an albumin) are typically treated to form a crosslinked matrix (e.g. by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. Patent Application Serial Number 10/335,506 and PCT publication WO 03/035891 which are incorporated herein by reference. For example, an amine cross-linking reagent, such as, but not limited to, glutaraldehyde, can be added to the protein mixture.

### PROTEIN CONSTITUENT

The electrochemical sensors of the invention optionally include a protein constituent disposed between the analyte sensing constituent and the analyte modulating constituent (see, e.g. element 116 in FIG. 2A). The term "protein constituent" is used herein according to art accepted terminology and refers to constituent containing a carrier protein or the like that is selected for compatibility with the analyte sensing constituent and/or the analyte modulating constituent. In typical embodiments, the protein constituent comprises an albumin such as human serum albumin. The HSA concentration may vary between about 0.5%-30% (w/v). Typically the HSA concentration is about 1-10% w/v, and most typically is about 5% w/v. In alternative embodiments of the invention, collagen or BSA or other structural proteins used in these contexts can be used instead of or in addition to HSA. This constituent is typically crosslinked on the analyte sensing constituent according to art accepted protocols.

### ADHESION PROMOTING CONSTITUENT

The electrochemical sensors of the invention can include one or more adhesion promoting (AP) constituents (see, e.g. element 114 in FIG. 2A). The term "adhesion promoting constituent" is used herein according to art accepted terminology and refers to a constituent that includes materials selected for their ability to promote adhesion between adjoining constituents in the sensor. Typically, the adhesion promoting constituent is disposed between the analyte sensing constituent and the analyte modulating constituent. Typically, the adhesion promoting constituent is disposed between the optional protein constituent and the analyte modulating constituent. The adhesion promoter constituent can be made from any one of a wide variety of materials known in the art to facilitate the bonding between such constituents and can be applied by any one of a wide variety of methods known in the art. Typically, the adhesion promoter constituent comprises a silane compound such as γ-aminopropyltrimethoxysilane.

The use of silane coupling reagents, especially those of the formula R'Si(OR)₃ in which R' is typically an aliphatic group with a terminal amine and R is a lower alkyl group, to promote adhesion is known in the art (see, e.g. U.S. Patent No. 5,212,050 which is incorporated herein by reference). For example, chemically modified electrodes in which a silane such as γ-aminopropyltriethoxysilane and glutaraldehyde were used in a step-wise process to attach and to co-crosslink bovine serum albumin (BSA) and glucose oxidase (GOₓ) to the electrode surface are well known in the art (see, e.g. Yao, T. Analytica Chim. Acta 1983, 148, 27-33).

In certain embodiments of the invention, the adhesion promoting constituent further comprises one or more compounds that can also be present in an adjacent constituent such as the polydimethyl siloxane (PDMS) compounds that serves to limit the diffusion of analytes such as glucose through the analyte modulating constituent. In illustrative embodiments the formulation comprises 0.5-20% PDMS, typically 5-15% PDMS, and most typically 10% PDMS. In certain embodiments of the invention, the adhesion promoting constituent is crosslinked within the layered sensor system and correspondingly includes an agent selected for its ability to crosslink a moiety present in a proximal constituent such as the analyte modulating constituent. In illustrative embodiments of the invention, the adhesion promoting constituent includes an agent selected for its ability to crosslink an amine or carboxyl moiety of a protein present in a proximal constituent such a the analyte sensing constituent and/or the protein constituent and or a siloxane moiety present in a compound disposed in a proximal layer such as the analyte modulating layer.

### ANALYTE MODULATING CONSTITUENT

The electrochemical sensors of the invention include an analyte modulating constituent disposed on the sensor (see, e.g. element 112 in FIG. 2A). Typically, the analyte modulating constituent comprises acrylate hydrogel compositions comprising bioactive agents as disclosed herein. The term "analyte modulating constituent" is used herein according to art accepted terminology and refers to a constituent that typically forms a membrane on the sensor that operates to modulate the diffusion of one or more analytes, such as glucose, through the constituent. In certain embodiments of the invention, the analyte modulating constituent is an analyte-limiting membrane (e.g. a glucose limiting membrane) which operates to prevent or restrict the diffusion of one or more analytes, such as glucose, through the constituents. In other embodiments of the invention, the analyte-modulating constituent operates to facilitate the diffusion of one or more analytes, through the constituents. Optionally such analyte modulating constituents can be formed to prevent or restrict the diffusion of one type of molecule through the constituent (e.g. glucose), while at the same time allowing or even facilitating the diffusion of other types of molecules through the constituent (e.g. O₂).

With respect to glucose sensors, in known enzyme electrodes, glucose and oxygen from blood, as well as some interferents, such as ascorbic acid and uric acid, diffuse through a primary membrane of the sensor. As the glucose, oxygen and interferents reach the analyte sensing constituent, an enzyme, such as glucose oxidase, catalyzes the conversion of glucose to hydrogen peroxide and gluconolactone. The hydrogen peroxide may diffuse back through the analyte modulating constituent, or it may diffuse to an electrode where it can be reacted to form oxygen and a proton to produce a current that is proportional to the glucose concentration. The sensor membrane assembly serves several functions, including selectively allowing the passage of glucose therethrough. In this context, an illustrative analyte modulating constituent is a semi-permeable membrane which permits passage of water, oxygen and at least one selective analyte and which has the ability to absorb water, the membrane having a water soluble, hydrophilic polymer.

A variety of illustrative analyte modulating compositions are known in the art and are described for example in U.S. Patent Nos. 6,319,540, 5,882,494, 5,786,439 5,777,060, 5,771,868 and 5,391,250, the disclosures of each being incorporated herein by reference. The hydrogels described therein are particularly useful with a variety of implantable devices for which it is advantageous to provide a surrounding water constituent.

### COVER CONSTITUENT

The electrochemical sensors of the invention include one or more cover constituents which are typically electrically insulating protective constituents (see, e.g. element 106 in FIG. 2A). Typically, such cover constituents can be in the form of a coating, sheath or tube and are disposed on at least a portion of the analyte modulating constituent. Acceptable polymer coatings for use as the insulating protective cover constituent can include, but are not limited to, non-toxic biocompatible polymers such as silicone compounds, polyimides, biocompatible solder masks, epoxy acrylate copolymers, or the like. Further, these coatings can be photo-imageable to facilitate photolithographic forming of apertures through to the conductive constituent. A typical cover constituent comprises spun on silicone. As is known in the art, this constituent can be a commercially available RTV (room temperature vulcanized) silicone composition. A typical chemistry in this context is polydimethyl siloxane (acetoxy based).

### ILLUSTRATIVE EMBODIMENTS OF ANALYTE SENSOR APPARATUS AND ASSOCIATED CHARACTERISTICS

The analyte sensor apparatus disclosed herein has a number of embodiments. A general embodiment of the invention is an analyte sensor apparatus for implantation within a mammal. While the analyte sensors are typically designed to be implantable within the body of a mammal, the sensors are not limited to any particular environment and can instead be used in a wide variety of contexts, for example for the analysis of most liquid samples including biological fluids such as whole-blood, lymph, plasma, serum, saliva, urine, stool, perspiration, mucus, tears, cerebrospinal fluid, nasal secretion, cervical or vaginal secretion, semen, pleural fluid, amniotic fluid, peritoneal fluid, middle ear fluid, joint fluid, gastric aspirate or the like. In addition, solid or desiccated samples may be dissolved in an appropriate solvent to provide a liquid mixture suitable for analysis.

As noted above, the sensor embodiments disclosed herein can be used to sense analytes of interest in one or more physiological environments. In certain embodiments for example, the sensor can be in direct contact with interstitial fluids as typically occurs with subcutaneous sensors. The sensors of the present invention may also be part of a skin surface system where interstitial glucose is extracted through the skin and brought into contact with the sensor (see, e.g. U.S. Patent Nos. 6,155,992 and 6,706,159 which are incorporated herein by reference). In other embodiments, the sensor can be in contact with blood as typically occurs for example with intravenous sensors. The sensor embodiments of the invention further include those adapted for use in a variety of contexts. In certain embodiments for example, the sensor can be designed for use in mobile contexts, such as those employed by ambulatory users. Alternatively, the sensor can be designed for use in stationary contexts such as those adapted for use in clinical settings. Such sensor embodiments include, for example, those used to monitor one or more analytes present in one or more physiological environments in a hospitalized patient.

Sensors of the invention can also be incorporated into a wide variety of medical systems known in the art. Sensors of the invention can be used, for example, in a closed loop infusion system designed to control the rate that medication is infused into the body of a user. Such a closed loop infusion system can include a sensor and an associated meter which generates an input to a controller which in turn operates a delivery system (e.g. one that calculates a dose to be delivered by a medication infusion pump). In such contexts, the meter associated with the sensor may also transmit commands to, and be used to remotely control, the delivery system. Typically, the sensor is a subcutaneous sensor in contact with interstitial fluid to monitor the glucose concentration in the body of the user, and the liquid infused by the delivery system into the body of the user includes insulin. Illustrative systems are disclosed for example in U. S. Patent Nos. 6,558,351 and 6,551,276; PCT Application Nos. US99/21703 and US99/22993; as well as WO 2004/008956 and WO 2004/009161, all of which are incorporated herein by reference.

### PERMUTATIONS OF ANALYTE SENSOR APPARATUS AND ELEMENTS

As noted above, the invention disclosed herein includes a number of embodiments including sensors having constellations of elements including acrylate hydrogel compositions comprising bioactive agents. Such embodiments of the invention allow artisans to generate a variety of permutations of the analyte sensor apparatus disclosed herein. As noted above, illustrative general embodiments of the sensor disclosed herein include a base layer, a cover layer and at least one layer having a sensor element such as an electrode disposed between the base and cover layers. Typically, an exposed portion of one or more sensor elements (e.g., a working electrode, a counter electrode, reference electrode, etc.) is coated with a very thin layer of material having an appropriate electrode chemistry. For example, an enzyme such as lactate oxidase, glucose oxidase, glucose dehydrogenase or hexokinase, can be disposed on the exposed portion of the sensor element within an opening or aperture defined in the cover layer. FIG. 2A illustrates a cross-section of a typical sensor structure 100 of the present invention. The sensor is formed from a plurality of layers of various conductive and non-conductive constituents disposed on each other according to a method of the invention to produce a sensor structure 100.

As noted above, in the sensors of the invention, the various layers (e.g. the analyte sensing layer) of the sensors can have one or more bioactive and/or inert materials incorporated therein. The term "incorporated" as used herein is meant to describe any state or condition by which the material incorporated is held on the outer surface of or within a solid phase or supporting matrix of the layer. Thus, the material "incorporated" may, for example, be immobilized, physically entrapped, attached covalently to functional groups of the matrix layer(s). Furthermore, any process, reagents, additives, or molecular linker agents which promote the "incorporation" of said material may be employed if these additional steps or agents are not detrimental to, but are consistent with the objectives of the present invention. This definition applies, of course, to any of the embodiments of the present invention in which a bioactive molecule (e.g. an enzyme such as glucose oxidase) is "incorporated." For example, certain layers of the sensors disclosed herein include a proteinaceous substance such as albumin which serves as a crosslinkable matrix. As used herein, a proteinaceous substance is meant to encompass substances which are generally derived from proteins whether the actual substance is a native protein, an inactivated protein, a denatured protein, a hydrolyzed species, or a derivatized product thereof. Examples of suitable proteinaceous materials include, but are not limited to enzymes such as glucose oxidase and lactate oxidase and the like, albumins (e.g. human serum albumin, bovine serum albumin etc.), caseins, gamma-globulins, collagens and collagen derived products (e.g., fish gelatin, fish glue, animal gelatin, and animal glue).

An illustrative embodiment of the invention is shown in FIG. 2A. This embodiment includes an electrically insulating base layer 102 to support the sensor 100. The electrically insulating layer base 102 can be made of a material such as a ceramic substrate, which may be self-supporting or further supported by another material as is known in the art. In an alternative embodiment, the electrically insulating layer 102 comprises a polyimide substrate, for example a polyimide tape, dispensed from a reel. Providing the layer 102 in this form can facilitate clean, high density mass production. Further, in some production processes using such a polyimide tape, sensors 100 can be produced on both sides of the tape.

Typical embodiments of the invention include an analyte sensing layer disposed on the base layer 102. In an illustrative embodiment as shown in FIG. 2A the analyte sensing layer comprises a conductive layer 104 which is disposed on insulating base layer 102. Typically the conductive layer 104 comprises one or more electrodes. The conductive layer 104 can be applied using many known techniques and materials as will be described hereafter, however, the electrical circuit of the sensor 100 is typically defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating protective cover layer 106 such as a polymer coating is typically disposed on portions of the conductive layer 104. Acceptable polymer coatings for use as the insulating protective layer 106 can include, but are not limited to, non-toxic biocompatible polymers such as polyimide, biocompatible solder masks, epoxy acrylate copolymers, or the like. Further, these coatings can be photo-imageable to facilitate photolithographic forming of apertures 108 through to the conductive layer 104. In certain embodiments of the invention, an analyte sensing layer is disposed upon a porous metallic and/or ceramic and/or polymeric matrix with this combination of elements functioning as an electrode in the sensor.

In the sensors of the present invention, one or more exposed regions or apertures 108 can be made through the protective layer 106 to the conductive layer 104 to define the contact pads and electrodes of the sensor 100. In addition to photolithographic development, the apertures 108 can be formed by a number of techniques, including laser ablation, chemical milling or etching or the like. A secondary photoresist can also be applied to the cover layer 106 to define the regions of the protective layer to be removed to form the apertures 108. An operating sensor 100 typically includes a plurality of electrodes such as a working electrode and a counter electrode electrically isolated from each other, however typically situated in close proximity to one another. Other embodiments may also include a reference electrode. Still other embodiments may utilize a separate reference element not formed on the sensor. The exposed electrodes and/or contact pads can also undergo secondary processing through the apertures 108, such as additional plating processing, to prepare the surfaces and/or strengthen the conductive regions.

An analyte sensing layer 110 is typically disposed on one or more of the exposed electrodes of the conductive layer 104 through the apertures 108. Typically, the analyte sensing layer 110 is a sensor chemistry layer and most typically an enzyme layer. Typically, the analyte sensing layer 110 comprises the enzyme glucose oxidase or the enzyme lactate oxidase. In such embodiments, the analyte sensing layer 110 reacts with glucose to produce hydrogen peroxide which modulates a current to the electrode which can be monitored to measure an amount of glucose present. The sensor chemistry layer 110 can be applied over portions of the conductive layer or over the entire region of the conductive layer. Typically the sensor chemistry layer 110 is disposed on portions of a working electrode and a counter electrode that comprise a conductive layer. Some methods for generating the thin sensor chemistry layer 110 include spin coating processes, dip and dry processes, low shear spraying processes, ink-jet printing processes, silk screen processes and the like. Most typically the thin sensor chemistry layer 110 is applied using a spin coating process.

The analyte sensing layer 110 is typically coated with one or more coating layers. In some embodiments of the invention, one such coating layer includes a membrane which can regulate the amount of analyte that can contact an enzyme of the analyte sensing layer. For example, a coating layer can comprise an analyte modulating membrane layer such as a glucose limiting membrane which regulates the amount of glucose that contacts the glucose oxidase enzyme layer on an electrode. Such glucose limiting membranes can be made from a wide variety of materials known to be suitable for such purposes, e.g., silicone, polyurethane, polyurea cellulose acetate, Nafion, polyester sulfonic acid (Kodak AQ), hydrogels or any other membrane known to those skilled in the art. In certain embodiments of the invention, the analyte modulating layer comprises a linear polyurethane/polyurea polymer polycarbonate with a branched acrylate hydrophilic comb-copolymer having a central chain and a plurality of side chains coupled to the central chain, wherein at least one side chain comprises a silicone moiety.

In some embodiments of the invention, a coating layer is a glucose limiting membrane layer 112 which is disposed above the sensor chemistry layer 110 to regulate glucose contact with the sensor chemistry layer 110. In some embodiments of the invention, an adhesion promoter layer 114 is disposed between the membrane layer 112 and the sensor chemistry layer 110 as shown in FIG. 2A in order to facilitate their contact and/or adhesion. The adhesion promoter layer 114 can be made from any one of a wide variety of materials known in the art to facilitate the bonding between such layers. Typically, the adhesion promoter layer 114 comprises a silane compound. In alternative embodiments, protein or like molecules in the sensor chemistry layer 110 can be sufficiently crosslinked or otherwise prepared to allow the membrane layer 112 to be disposed in direct contact with the sensor chemistry layer 110 in the absence of an adhesion promoter layer 114.

As noted above, embodiments of the present invention can include one or more functional coating layers. As used herein, the term "functional coating layer" denotes a layer that coats at least a portion of at least one surface of a sensor, more typically substantially all of a surface of the sensor, and that is capable of interacting with one or more analytes, such as chemical compounds, cells and fragments thereof, etc., in the environment in which the sensor is disposed. Non-limiting examples of functional coating layers include sensor chemistry layers (e.g., enzyme layers), analyte limiting layers, biocompatible layers; layers that increase the slipperiness of the sensor; layers that promote cellular attachment to the sensor; layers that reduce cellular attachment to the sensor; and the like. Typically analyte modulating layers operate to prevent or restrict the diffusion of one or more analytes, such as glucose, through the layers. Optionally such layers can be formed to prevent or restrict the diffusion of one type of molecule through the layer (e.g. glucose), while at the same time allowing or even facilitating the diffusion of other types of molecules through the layer (e.g. O₂). An illustrative functional coating layer is a hydrogel such as those disclosed in U.S. Patent Nos. 5,786,439 and 5,391,250, the disclosures of each being incorporated herein by reference. The hydrogels described therein are particularly useful with a variety of implantable devices for which it is advantageous to provide a surrounding water layer.

The sensor embodiments disclosed herein can include layers having UV-absorbing polymers. In accordance with one aspect of the present invention, there is provided a sensor including at least one functional coating layer including an UV-absorbing polymer. In some embodiments, the UV-absorbing polymer is a polyurethane, a polyurea or a polyurethane/polyurea copolymer. More typically, the selected UV-absorbing polymer is formed from a reaction mixture including a diisocyanate, at least one diol, diamine or mixture thereof, and a polyfunctional UV-absorbing monomer.

UV-absorbing polymers are used with advantage in a variety of sensor fabrication methods, such as those described in U.S. Pat. No. 5,390,671, to Lord et al., entitled "Transcutaneous Sensor Insertion Set"; No. 5,165,407, to Wilson et al., entitled "Implantable Glucose Sensor"; and U.S. Pat. No. 4,890,620, to Gough, entitled "Two-Dimensional Diffusion Glucose Substrate Sensing Electrode", which are incorporated herein in their entireties by reference. However, any sensor production method which includes the step of forming an UV-absorbing polymer layer above or below a sensor element is considered to be within the scope of the present invention. In particular, the inventive methods are not limited to thin-film fabrication methods, and can work with other sensor fabrication methods that utilize UV-laser cutting. Embodiments can work with thick-film, planar or cylindrical sensors and the like, and other sensor shapes requiring laser cutting.

As disclosed herein, the sensors of the present invention are particularly designed for use as subcutaneous or transcutaneous glucose sensors for monitoring blood glucose levels in a diabetic patient. Typically each sensor comprises a plurality of sensor elements, for example electrically conductive elements such as elongated thin film conductors, formed between an underlying insulative thin film base layer and an overlying insulative thin film cover layer.

If desired, a plurality of different sensor elements can be included in a single sensor. For example, both conductive and reactive sensor elements can be combined in one sensor, optionally with each sensor element being disposed on a different portion of the base layer. One or more control elements can also be provided. In such embodiments, the sensor can have defined in its cover layer a plurality of openings or apertures. One or more openings can also be defined in the cover layer directly over a portion of the base layer, in order to provide for interaction of the base layer with one or more analytes in the environment in which the sensor is disposed. The base and cover layers can be comprised of a variety of materials, typically polymers. In more specific embodiments the base and cover layers are comprised of an insulative material such as a polyimide. Openings are typically formed in the cover layer to expose distal end electrodes and proximal end contact pads. In a glucose monitoring application, for example, the sensor can be placed trans cutaneously so that the distal end electrodes are in contact with patient blood or extracellular fluid, and the contact pads are disposed externally for convenient connection to a monitoring device.

### ILLUSTRATIVE METHODS AND MATERIALS FOR MAKING ANALYTE SENSOR APPARATUS OF THE INVENTION

A number of articles, U.S. patents and patent application describe the state of the art with the common methods and materials disclosed herein and further describe various elements (and methods for their manufacture) that can be used in the sensor designs disclosed herein. These include for example, U.S. Patent Nos. 6,413,393; 6,368,274; 5,786,439; 5,777,060; 5,391,250; 5,390,671; 5,165,407, 4,890,620, 5,390,671, 5,390,691, 5,391,250, 5,482,473, 5,299,571, 5,568,806; United States Patent Application 20020090738; as well as PCT International Publication Numbers WO 01/58348, WO 03/034902, WO 03/035117, WO 03/035891, WO 03/023388, WO 03/022128, WO 03/022352, WO 03/023708, WO 03/036255, WO03/036310 and WO 03/074107, the contents of each of which are incorporated herein by reference.

Typical sensors for monitoring glucose concentration of diabetics are further described in Shichiri, et al., "In Vivo Characteristics of Needle-Type Glucose Sensor-Measurements of Subcutaneous Glucose Concentrations in Human Volunteers," Horm. Metab. Res., Suppl. Ser. 20:17-20 (1988); Bruckel, et al.,: "In Vivo Measurement of Subcutaneous Glucose Concentrations with an Enzymatic Glucose Sensor and a Wick Method," Klin. Wochenschr. 67:491-495 (1989); and Pickup, et al.,: "In Vivo Molecular Sensing in Diabetes Mellitus: An Implantable Glucose Sensor with Direct Electron Transfer," Diabetologia 32:213-217 (1989). Other sensors are described in, for example Reach, et al., in ADVANCES IN IMPLANTABLE DEVICES, A. Turner (ed.), JAI Press, London, Chap. 1, (1993), incorporated herein by reference.

Electrodes of the invention can be formed from a wide variety of materials known in the art. For example, the electrode may be made of a noble late transition metals. Metals such as gold, platinum, silver, rhodium, iridium, ruthenium, palladium, or osmium can be suitable in various embodiments of the invention. Other compositions such as carbon or mercury can also be useful in certain sensor embodiments. Of these metals, silver, gold, or platinum is typically used as a reference electrode metal. A silver electrode which is subsequently chloridized is typically used as the reference electrode. These metals can be deposited by any means known in the art, including the plasma deposition method cited, supra, or by an electroless method which may involve the deposition of a metal onto a previously metallized region when the substrate is dipped into a solution containing a metal salt and a reducing agent. The electroless method proceeds as the reducing agent donates electrons to the conductive (metallized) surface with the concomitant reduction of the metal salt at the conductive surface. The result is a layer of adsorbed metal. (For additional discussions on electroless methods, see: Wise, E. M. Palladium: Recovery, Properties, and Uses, Academic Press, New York, New York (1988); Wong, K. et al. Plating and Surface Finishing 1988, 75, 70-76; Matsuoka, M. et al. Ibid. 1988, 75, 102-106; and Pearlstein, F. "Electroless Plating," Modern Electroplating, Lowenheim, F. A., Ed., Wiley, New York, N.Y. (1974), Chapter 31.). Such a metal deposition process must yield a structure with good metal to metal adhesion and minimal surface contamination, however, to provide a catalytic metal electrode surface with a high density of active sites. Such a high density of active sites is a property necessary for the efficient redox conversion of an electroactive species such as hydrogen peroxide.

In an exemplary embodiment of the invention, the base layer is initially coated with a thin film conductive layer by electrode deposition, surface sputtering, or other suitable process step. In one embodiment this conductive layer may be provided as a plurality of thin film conductive layers, such as an initial chrome-based layer suitable for chemical adhesion to a polyimide base layer followed by subsequent formation of thin film gold-based and chrome-based layers in sequence. In alternative embodiments, other electrode layer conformations or materials can be used. The conductive layer is then covered, in accordance with conventional photolithographic techniques, with a selected photoresist coating, and a contact mask can be applied over the photoresist coating for suitable photoimaging. The contact mask typically includes one or more conductor trace patterns for appropriate exposure of the photoresist coating, followed by an etch step resulting in a plurality of conductive sensor traces remaining on the base layer. In an illustrative sensor construction designed for use as a subcutaneous glucose sensor, each sensor trace can include three parallel sensor elements corresponding with three separate electrodes such as a working electrode, a counter electrode and a reference electrode.

Portions of the conductive sensor layers are typically covered by an insulative cover layer, typically of a material such as a silicon polymer and/or a polyimide. The insulative cover layer can be applied in any desired manner. In an exemplary procedure, the insulative cover layer is applied in a liquid layer over the sensor traces, after which the substrate is spun to distribute the liquid material as a thin film overlying the sensor traces and extending beyond the marginal edges of the sensor traces in sealed contact with the base layer. This liquid material can then be subjected to one or more suitable radiation and/or chemical and/or heat curing steps as are known in the art. In alternative embodiments, the liquid material can be applied using spray techniques or any other desired means of application. Various insulative layer materials may be used such as photoimagable epoxyacrylate, with an illustrative material comprising a photoimagable polyimide available from OCG, Inc. of West Paterson, N.J., under the product number 7020.

As noted above, appropriate electrode chemistries defining the distal end electrodes can be applied to the sensor tips, optionally subsequent to exposure of the sensor tips through the openings. In an illustrative sensor embodiment having three electrodes for use as a glucose sensor, an enzyme (typically glucose oxidase) is provided within one of the openings, thus coating one of the sensor tips to define a working electrode. One or both of the other electrodes can be provided with the same coating as the working electrode. Alternatively, the other two electrodes can be provided with other suitable chemistries, such as other enzymes, left uncoated, or provided with chemistries to define a reference electrode and a counter electrode for the electrochemical sensor.

Methods for producing the extremely thin enzyme coatings of the invention include spin coating processes, dip and dry processes, low shear spraying processes, ink-jet printing processes, silk screen processes and the like. As artisans can readily determine the thickness of an enzyme coat applied by process of the art, they can readily identify those methods capable of generating the extremely thin coatings of the invention. Typically, such coatings are vapor crosslinked subsequent to their application. Surprisingly, sensors produced by these processes have material properties that exceed those of sensors having coatings produced by electrodeposition including enhanced longevity, linearity, regularity as well as improved signal to noise ratios. In addition, embodiments of the invention that utilize glucose oxidase coatings formed by such processes are designed to recycle hydrogen peroxide and improve the biocompatibility profiles of such sensors.

Sensors generated by processes such as spin coating processes also avoid other problems associated with electrodeposition, such as those pertaining to the material stresses placed on the sensor during the electrodeposition process. In particular, the process of electrodeposition is observed to produce mechanical stresses on the sensor, for example mechanical stresses that result from tensile and/or compression forces. In certain contexts, such mechanical stresses may result in sensors having coatings with some tendency to crack or delaminate. This is not observed in coatings disposed on sensor via spin coating or other low-stress processes. Consequently, yet another embodiment of the invention is a method of avoiding the electrodeposition influenced cracking and/or delamination of a coating on a sensor comprising applying the coating via a spin coating process.

### METHODS FOR USING ANALYTE SENSOR APPARATUS OF THE INVENTION

A related embodiment of the invention is a method of sensing an analyte within the body of a mammal, the method comprising implanting an analyte sensor embodiment disclosed herein in to the mammal and then sensing an alteration in current at the working electrode and correlating the alteration in current with the presence of the analyte, so that the analyte is sensed. The analyte sensor can polarized anodically such that the working electrode where the alteration in current is sensed is an anode, or cathodically such that the working electrode where the alteration in current is sensed is a cathode. In one such method, the analyte sensor apparatus senses glucose in the mammal. In an alternative method, the analyte sensor apparatus senses lactate, potassium, calcium, oxygen, pH, and/or any physiologically relevant analyte in the mammal.

Certain analyte sensors having the analyte modulating compositions comprising an bioactive agent and the structures discussed above have a number of highly desirable characteristics which allow for a variety of methods for sensing analytes in a mammal. For example, in such methods, the analyte sensor apparatus implanted in the mammal functions to sense an analyte within the body of a mammal for more than 1, 2, 3, 4, 5, or 6 weeks. Typically, the analyte sensor apparatus so implanted in the mammal senses an alteration in current in response to an analyte within 15, 10, 5 or 2 minutes of the analyte contacting the sensor. In such methods, the sensors can be implanted into a variety of locations within the body of the mammal, for example interstitially, as well as in both vascular and other non-vascular spaces.

Further disclosed herein is the subject-matter of the following clauses:
1. An amperometric analyte sensor comprising:
   a working electrode comprising:
   a base layer;
   a conductive layer disposed over the base layer;
   an analyte sensing layer disposed over the conductive layer; and
   an analyte modulating layer disposed over the analyte sensing layer, wherein:
      the analyte modulating layer permselectively modulates the diffusion of glucose and oxygen therethrough such that the diffusion of glucose is limited relative to oxygen; and
      the analyte modulating layer comprises an acrylate hydrogel having a polymer reversibly coupled to a bioactive agent such that the bioactive agent is uncoupled from the polymer in response to a stimuli.
2. The amperometric analyte sensor of clause 1, wherein:
   the bioactive agent is at least one of: an antibacterial agent, an anti-inflammatory agent and an anticoagulant agent; and/or
   the analyte modulating layer exhibits a permeability to glucose and oxygen that is altered by less than 10% following release of the bioactive agent from the analyte modulating layer.
3. The amperometric analyte sensor of clause 1 or 2, wherein the bioactive agent comprises at least one of a dexamethasone, a heparin or a fluoroquinolone.
4. The amperometric analyte sensor of clause 2 or of any of the clauses 1 to 3, wherein;
   the bioactive agent is noncovalently entrapped within the polymer; and/or
   the bioactive agent is covalently coupled to the polymer; and/or
   the bioactive agent is coupled to the polymer by an acrylate moiety disposed on the bioactive agent.
5. The amperometric analyte sensor of clause 1 or of any of the clauses 1 to 4, wherein the analyte modulating layer comprises at least one of: a Poly(2-hydroxyethyl methacrylate), a polyurethane and a chain extender.
6. The amperometric analyte sensor of clause 1 or of any of the clauses 1 to 5, wherein the bioactive agent is uncoupled from the polymer in response to:
   exposure to aqueous media;
   exposure to endogenous stimuli present in the environment in which the sensor is disclosed;
   an alteration in the pH of the environment in which the amperometric analyte sensor is disposed;
   an alteration in the temperature of the environment in which the amperometric analyte sensor is disposed; and/or
   an electrochemical stimuli selected from: a voltage applied to the amperometric analyte sensor; and/or a current within the amperometric analyte sensor.
7. The amperometric analyte sensor of clause 6 or of any of the clauses 1 to 6, wherein the bioactive agent is uncoupled from the polymer in response to: active oxygen species including hydrogen peroxide.
8. The amperometric analyte sensor of clause 1 or of any of the clauses 1 to 7, wherein the amperometric analyte sensor consists of a single sensor flex assembly comprising a flexible planar element having a longitudinal member comprising a first side and a second side.
9. The amperometric analyte sensor of clause 1 or of any of the clauses 1 to 8, wherein the bioactive agent is coupled to an external surface of the analyte modulating layer.
10. A method of making an amperometric analyte sensor electrode for implantation within a mammal comprising the steps of:
   forming a working electrode comprising:
   a base layer;
   a conductive layer disposed on the base layer;
   an analyte sensing layer disposed on the conductive layer;
   an analyte modulating layer disposed on the analyte sensing layer, wherein:
      the analyte modulating layer is formed from materials selected to permselectively modulate the diffusion of glucose and oxygen therethrough such that the diffusion of glucose is limited relative to oxygen; and
      the analyte modulating layer is formed to comprise an acrylate hydrogel having a polymer reversibly coupled to a bioactive agent such that the bioactive agent is uncoupled from the polymer in response to a stimuli.
11. The method of clause 10, wherein the method comprises forming the sensor electrode from materials selected to modulate the hydrophilicity of the analyte modulating layer when the sensor electrode is disposed in an interstitial space.
12. The method of clause 10 or 11, wherein the analyte modulating layer is formed from materials selected so that the bioactive agent is uncoupled from the polymer in response to:
   exposure to aqueous media;
   exposure to glucose;
   an alteration in the pH of the environment in which the amperometric analyte sensor is disposed;
   an alteration in the temperature of the environment in which the amperometric analyte sensor is disposed; and/or
   an electrochemical stimuli selected from: a voltage applied to the amperometric analyte sensor; and/or a current within the amperometric analyte sensor.
13. The method of clause 10 or of any of the clauses 10 to 12, wherein the bioactive agent is selected to be at least one of: an antibacterial agent, an anti-inflammatory agent and an anticoagulant agent.
14. The method of clause 10 or of any of the clauses 10 to 13, wherein the bioactive agent comprises at least one of a dexamethasone, a heparin or a fluoroquinolone.
15. The method of clause 10 or of any of the clauses 10 to 14, wherein the bioactive agent is formed to comprise an acrylate moiety which couples the bioactive agent to the polymer.
16. The method of clauses 10 or of any of the clauses 10 to 15, wherein the analyte modulating layer comprises at least one of: a Poly(2-hydroxyethyl methacrylate), a polyurethane and a chain extender.
17. The method of clause 10 or of any of the clauses 10 to 16, wherein the bioactive agent is coupled to an external surface of the analyte modulating layer.
18. The method of clause 10 or of any of the clauses 10 to 17, wherein the analyte modulating layer is formed from a reaction mixture comprising a photoactive agent selected to facilitate polymerization.
19. The method of clause 10 or of any of the clauses 10 to 18, further comprising performing a sterilization step on the sensor electrode, wherein the sterilization step comprises exposure to ethylene oxide.
20. A method of sensing an analyte within the body of a mammal using an electrochemical analyte sensor while simultaneously inhibiting a foreign body response to the electrochemical analyte sensor, the method comprising:
   implanting an electrochemical analyte sensor of claim 1 into the mammal;
   sensing an alteration in current at the working electrode in the presence of the analyte; and correlating the alteration in current with the presence of the analyte, so that the analyte is sensed.

## Claims

1. An amperometric analyte sensor comprising:
a working electrode comprising:
a base layer;
a conductive layer disposed over the base layer;
an analyte sensing layer disposed over the conductive layer; and
an analyte modulating layer disposed over the analyte sensing layer, wherein:
the analyte modulating layer permselectively modulates the diffusion of glucose and oxygen therethrough such that the diffusion of glucose is limited relative to oxygen; and
the analyte modulating layer comprises an acrylate hydrogel having a polymer reversibly coupled to a bioactive agent such that the bioactive agent is uncoupled from the polymer in response to a stimuli.

2. The amperometric analyte sensor of claim 1, wherein:
the bioactive agent is at least one of: an antibacterial agent, an anti-inflammatory agent and an anticoagulant agent; and/or
the analyte modulating layer exhibits a permeability to glucose and oxygen that is altered by less than 10% following release of the bioactive agent from the analyte modulating layer.

3. The amperometric analyte sensor of claim 1 or 2, wherein the bioactive agent comprises at least one of a dexamethasone, a heparin or a fluoroquinolone.

4. The amperometric analyte sensor of any of the claims 1 to 3, wherein;
the bioactive agent is noncovalently entrapped within the polymer; and/or
the bioactive agent is covalently coupled to the polymer; and/or
the bioactive agent is coupled to the polymer by an acrylate moiety disposed on the bioactive agent.

5. The amperometric analyte sensor of any of the claims 1 to 4, wherein the analyte modulating layer comprises at least one of: a Poly(2-hydroxyethyl methacrylate), a polyurethane and a chain extender.

6. The amperometric analyte sensor of any of the claims 1 to 5, wherein the bioactive agent is uncoupled from the polymer in response to:
exposure to aqueous media;
exposure to endogenous stimuli present in the environment in which the sensor is disclosed;
an alteration in the pH of the environment in which the amperometric analyte sensor is disposed;
an alteration in the temperature of the environment in which the amperometric analyte sensor is disposed; and/or
an electrochemical stimuli selected from: a voltage applied to the amperometric analyte sensor; and/or a current within the amperometric analyte sensor,
and/or
wherein the bioactive agent is uncoupled from the polymer in response to: active oxygen species including hydrogen peroxide.

7. The amperometric analyte sensor of any of the claims 1 to 6, wherein the amperometric analyte sensor consists of a single sensor flex assembly comprising a flexible planar element having a longitudinal member comprising a first side and a second side.

8. The amperometric analyte sensor of any of the claims 1 to 7, wherein the bioactive agent is coupled to an external surface of the analyte modulating layer.

9. A method of making an amperometric analyte sensor electrode for implantation within a mammal comprising the steps of:
forming a working electrode comprising:
a base layer;
a conductive layer disposed on the base layer;
an analyte sensing layer disposed on the conductive layer;
an analyte modulating layer disposed on the analyte sensing layer, wherein:
the analyte modulating layer is formed from materials selected to permselectively modulate the diffusion of glucose and oxygen therethrough such that the diffusion of glucose is limited relative to oxygen; and
the analyte modulating layer is formed to comprise an acrylate hydrogel having a polymer reversibly coupled to a bioactive agent such that the bioactive agent is uncoupled from the polymer in response to a stimuli.

10. The method of claim 9, wherein the method comprises forming the sensor electrode from materials selected to modulate the hydrophilicity of the analyte modulating layer when the sensor electrode is disposed in an interstitial space.

11. The method of claim 9 or 10, wherein the analyte modulating layer is formed from materials selected so that the bioactive agent is uncoupled from the polymer in response to:
exposure to aqueous media;
exposure to glucose;
an alteration in the pH of the environment in which the amperometric analyte sensor is disposed;
an alteration in the temperature of the environment in which the amperometric analyte sensor is disposed; and/or
an electrochemical stimuli selected from: a voltage applied to the amperometric analyte sensor; and/or a current within the amperometric analyte sensor.

12. The method of any of the claims 9 to 11, wherein the bioactive agent is selected to be at least one of: an antibacterial agent, an anti-inflammatory agent and an anticoagulant agent, and/or wherein the bioactive agent comprises at least one of a dexamethasone, a heparin or a fluoroquinolone, and/or
wherein the bioactive agent is formed to comprise an acrylate moiety which couples the bioactive agent to the polymer.

13. The method of any of the claims 9 to 12, wherein the analyte modulating layer comprises at least one of: a Poly(2-hydroxyethyl methacrylate), a polyurethane and a chain extender, and/or wherein the bioactive agent is coupled to an external surface of the analyte modulating layer, and/or wherein the analyte modulating layer is formed from a reaction mixture comprising a photoactive agent selected to facilitate polymerization.

14. The method of any of the claims 9 to 13, further comprising performing a sterilization step on the sensor electrode, wherein the sterilization step comprises exposure to ethylene oxide.

15. A method of sensing an analyte within the body of a mammal using an electrochemical analyte sensor while simultaneously inhibiting a foreign body response to the electrochemical analyte sensor, the method comprising:
implanting an electrochemical analyte sensor of claim 1 into the mammal;
sensing an alteration in current at the working electrode in the presence of the analyte; and correlating the alteration in current with the presence of the analyte, so that the analyte is sensed.
